# EUROPEAN PATENT SPECIFICATION

(11) **EP 1 124 979 B1**
(45) Date of publication and mention of the grant of the patent: **30.08.2006**
(21) Application number: 00959652.9
(22) Date of filing: 30.08.2000
(51) Int. Cl.: C12P 7/22, C12N 9/02, C12N 9/14, C12N 1/20, C12N 15/00

(54) **PRODUCTION OF 3-HYDROXYPROPIONIC ACID IN RECOMBINANT ORGANISMS**
HERSTELLUNG VON 3-HYDROXYPROPIONSÄURE IN REKOMBINANTEN ORGANISMEN
PRODUCTION D'ACIDE 3-HYDROXYPROPIONIQUE PAR DES ORGANISMES RECOMBINANTS

(30) Priority: 30.08.1999 US 151440 P
(43) Date of publication of application: 22.08.2001
(73) Proprietor: WISCONSIN ALUMNI RESEARCH FOUNDATION, Madison, WI 53707-7365 (US)
(72) Inventor: SUTHERS, Patrick, F., Madison, WI 53715 (US); CAMERON, Douglas, C., North Plymouth, MN 55447 (US)
(74) Representative: Duckworth, Timothy John
(86) International application number: PCT/US2000/023878
(87) International publication number: WO 2001/016346

(56) References cited:
- WO-A-00/08198
- WO-A-98/21339
- TONG I-T ET AL: "1,3-Propanediol production by Escherichia coli expressing genes from the Klebsiella pneumoniae dha regulon" APPLIED AND ENVIRONMENTAL MICROBIOLOGY, WASHINGTON,DC, US, vol. 57, 1 December 1991 (1991-12-01), pages 3541-3546, XP002062741 ISSN: 0099-2240
- TANG ET AL.: 'Immunochemical properties of NAD+ - linked glycerol dehydrogenases from escherichia coli and klebsiella pneumoniae' JOURNAL OF BACTERIOLOGY vol. 152, no. 3, December 1982, pages 1169 - 1174, XP002933455
- BARBIRATO ET AL.: 'Anaerobic pathways of glycerol dissimilation by enterobacter agglomerans CNCM 1210: Limitations and regulations' MICROBIOLOGY vol. 143, no. 7, 1997, pages 2423 - 2432, XP002933456

### SEQUENCE LISTING

<110> Wisconsin Alumni Research Foundation
<120> Production of 3-Hydroxypropionic Acid in Recombinant Organisms
<130> N.82478 PEJ/TJD
<140> EP 00959652.9
   <141> 2000-08-30
<150> PCT/US00/23878
   <151> 2000-08-30
<150> US 60/151440
   <151> 1999-08-30
<160> 23
<170> PatentIn Ver. 2.1
<210> 1
   <211> 1529
   <212> DNA
   <213> Saccharomyces cerevisiae
<220>
   <221> CDS
   <222> (25)..(1509)
<400> 1
   gtcgcggtac caaggaggta tcat atg tca cac ctt cct atg aca gtg cct     51
                              Met Ser His Leu Pro Met Thr Val Pro
                                1               5
   atc aag ctg ccc aat ggg ttg gaa tat gag caa cca acg ggg ttg ttc    99
   Ile Lys Leu Pro Asn Gly Leu Glu Tyr Glu Gln Pro Thr Gly Leu Phe
    10                  15                  20                  25
   atc aac aac aag ttt gtt cct tct aaa cag aac aag acc ttc gaa gtc   147
   Ile Asn Asn Lys Phe Val Pro Ser Lys Gln Asn Lys Thr Phe Glu Val
                    30                  35                  40
   att aac cct tcc acg gaa gaa gaa ata tgt cat att tat gaa ggt aga    195
   Ile Asn Pro Ser Thr Glu Glu Glu Ile Cys His Ile Tyr Glu Gly Arg
               45                  50                  55
   gag gac gat gtg gaa gag gcc gtg cag gcc gcc gac cgt gcc ttc tct    243
   Glu Asp Asp Val Glu Glu Ala Val Gln Ala Ala Asp Arg Ala Phe Ser
           60                  65                  70
   aat ggg tct tgg aac ggt atc gac cct att gac agg ggt aag gct ttg    291
   Asn Gly Ser Trp Asn Gly Ile Asp Pro Ile Asp Arg Gly Lys Ala Leu
       75                  80                  85
   tac agg tta gcc gaa tta att gaa cag gac aag gat gtc att gct tcc    339
   Tyr Arg Leu Ala Glu Leu Ile Glu Gln Asp Lys Asp Val Ile Ala Ser
   90                  95                  100                 105
   atc gag act ttg gat aac ggt aaa gct atc tct tcc tcg aga gga gat    387
   Ile Glu Thr Leu Asp Asn Gly Lys Ala Ile Ser Ser Ser Arg Gly Asp
                   110                 115                 120
   gtt gat tta gtc atc aac tat ttg aaa tct tcc gct ggc ttt gct gat    435
   Val Asp Leu Val Ile Asn Tyr Leu Lys Ser Ser Ala Gly Phe Ala Asp
               125                 130                 135
   aaa att gat ggt aga atg att gat act ggt aga acc cat ttt tct tac    483
   Lys Ile Asp Gly Arg Met Ile Asp Thr Gly Arg Thr His Phe Ser Tyr
           140                 145                 150
   act aag aga cag cct ttg ggt gtt tgt ggg cag att att cct tgg aat    531
   Thr Lys Arg Gln Pro Leu Gly Val Cys Gly Gln Ile Ile Pro Trp Asn
       155                 160                 165
   ttc cca ctg ttg atg tgg gcc tgg aag att gcc cct gct ttg gtc acc    579
   Phe Pro Leu Leu Met Trp Ala Trp Lys Ile Ala Pro Ala Leu Val Thr
   170                 175                 180                 185
   ggt aac acc gtc gtg ttg aag act gcc gaa tcc acc cca ttg tcc gct   627
   Gly Asn Thr Val Val Leu Lys Thr Ala Glu Ser Thr Pro Leu Ser Ala
                   190                 195                 200
   ttg tat gtg tct aaa tac atc cca cag gcg ggt att cca cct ggt gtg   675
   Leu Tyr Val Ser Lys Tyr Ile Pro Gln Ala Gly Ile Pro Pro Gly Val
               205                 210                 215
   atc aac att gta tcc ggg ttt ggt aag att gtg gtt gag gcc att aca   723
   Ile Asn Ile Val Ser Gly Phe Gly Lys Ile Val Val Glu Ala Ile Thr
           220                 225                 230
   aac cat cca aaa atc aaa aag gtt gcc ttc aca ggg tcc acg gct acg   771
   Asn His Pro Lys Ile Lys Lys Val Ala Phe Thr Gly Ser Thr Ala Thr
       235                 240                 245
   ggt aga cac att tac cag tcc gca gcc gca ggc ttg aaa aaa gtg act   819
   Gly Arg His Ile Tyr Gln Ser Ala Ala Ala Gly Leu Lys Lys Val Thr
   250                 255                 260                 265
   ttg gag ctg ggt ggt aaa tca cca aac att gtc ttc gcg gac gcc gag   867
   Leu Glu Leu Gly Gly Lys Ser Pro Asn Ile Val Phe Ala Asp Ala Glu
                   270                 275                 280
   ttg aaa aaa gcc gtg caa aac att atc ctt ggt atc tac tac aat tct   915
   Leu Lys Lys Ala Val Gln Asn Ile Ile Leu Gly Ile Tyr Tyr Asn Ser
               285                 290                 295
   ggt gag gtc tgt tgt gcg ggt tca agg gtg tat gtt gaa gaa tct att   963
   Gly Glu Val Cys Cys Ala Gly Ser Arg Val Tyr Val Glu Glu Ser Ile
           300                 305                 310
   tac gac aaa ttc att gaa gag ttc aaa gcc gct tct gaa tcc atc aag   1011
   Tyr Asp Lys Phe Ile Glu Glu Phe Lys Ala Ala Ser Glu Ser Ile Lys
       315                 320                 325
   gtg ggc gac cca ttc gat gaa tct act ttc caa ggt gca caa acc tct   1059
   Val Gly Asp Pro Phe Asp Glu Ser Thr Phe Gln Gly Ala Gln Thr Ser
   330                 335                 340                 345
   caa atg caa cta aac aaa atc ttg aaa tac gtt gac att ggt aag aat   1107
   Gln Met Gln Leu Asn Lys Ile Leu Lys Tyr Val Asp Ile Gly Lys Asn
                   350                 355                 360
   gaa ggt gct act ttg att acc ggt ggt gaa aga tta ggt agc aag ggt   1155
   Glu Gly Ala Thr Leu Ile Thr Gly Gly Glu Arg Leu Gly Ser Lys Gly
               365                 370                 375
   tac ttc att aag cca act gtc ttt ggt gac gtt aag gaa gac atg aga   1203
   Tyr Phe Ile Lys Pro Thr Val Phe Gly Asp Val Lys Glu Asp Met Arg
           380                 385                 390
   att gtc aaa gag gaa atc ttt ggc cct gtt gtc act gta acc aaa ttc   1251
   Ile Val Lys Glu Glu Ile Phe Gly Pro Val Val Thr Val Thr Lys Phe
       395                 400                 405
   aaa tct gcc gac gaa gtc att aac atg gcg aac gat tct gaa tac ggg   1299
   Lys Ser Ala Asp Glu Val Ile Asn Met Ala Asn Asp Ser Glu Tyr Gly
   410                 415                 420                 425
   ttg gct gct ggt att cac acc tct aat att aat acc gcc tta aaa gtg   1347
   Leu Ala Ala Gly Ile His Thr Ser Asn Ile Asn Thr Ala Leu Lys Val
                   430                 435                 440
   gct gat aga gtt aat gcg ggt acg gtc tgg ata aac act tat aac gat   1395
   Ala Asp Arg Val Asn Ala Gly Thr Val Trp Ile Asn Thr Tyr Asn Asp
               445                 450                 455
   ttc cac cac gca gtt cct ttc ggt ggg ttc aat gca tct ggt ttg ggc   1443
   Phe His His Ala Val Pro Phe Gly Gly Phe Asn Ala Ser Gly Leu Gly
           460                 465                 470
   agg gaa atg tct gtt gat gct tta caa aac tac ttg caa gtt aaa gcg 1491
   Arg Glu Met Ser Val Asp Ala Leu Gln Asn Tyr Leu Gln Val Lys Ala
   475 480 485
   gtc cgt gcc aaa ttg gac gagtaagagc tcgaattcgc 1529
   Val Arg Ala Lys Leu Asp
   490 495
<210> 2
   <211> 495
   <212> PRT
   <213> Saccharomyces cerevisiae.
<400> 2
   Met Ser His Leu Pro Met Thr Val Pro Ile Lys Leu Pro Asn Gly Leu
   1 5 10 15
   Glu Tyr Glu Gln Pro Thr Gly Leu Phe Ile Asn Asn Lys Phe Val Pro
   20 25 30
   Ser Lys Gln Asn Lys Thr Phe Glu Val Ile Asn Pro Ser Thr Glu Glu
   35 40 45
   Glu Ile Cys His Ile Tyr Glu Gly Arg Glu Asp Asp Val Glu Glu Ala
   50 55 60
   Val Gln Ala Ala Asp Arg Ala Phe Ser Asn Gly Ser Trp Asn Gly Ile
   65 70 75 80
   Asp Pro Ile Asp Arg Gly Lys Ala Leu Tyr Arg Leu Ala Glu Leu Ile
   85 90 95
   Glu Gln Asp Lys Asp Val Ile Ala Ser Ile Glu Thr Leu Asp Asn Gly
   100 105 110
   Lys Ala Ile Ser Ser Ser Arg Gly Asp Val Asp Leu Val Ile Asn Tyr
   115 120 125
   Leu Lys Ser Ser Ala Gly Phe Ala Asp Lys Ile Asp Gly Arg Met Ile
   130 135 140
   Asp Thr Gly Arg Thr His Phe Ser Tyr Thr Lys Arg Gln Pro Leu Gly
   145 150 155 160
   Val Cys Gly Gln Ile Ile Pro Trp Asn Phe Pro Leu Leu Met Trp Ala
   165 170 175
   Trp Lys Ile Ala Pro Ala Leu Val Thr Gly Asn Thr Val Val Leu Lys
   180 185 190
   Thr Ala Glu Ser Thr Pro Leu Ser Ala Leu Tyr Val Ser Lys Tyr Ile
   195 200 205
   Pro Gln Ala Gly Ile Pro Pro Gly Val Ile Asn Ile Val Ser Gly Phe
   210 215 220
   Gly Lys Ile Val Val Glu Ala Ile Thr Asn His Pro Lys Ile Lys Lys
   225 230 235 240
   Val Ala Phe Thr Gly Ser Thr Ala Thr Gly Arg His Ile Tyr Gln Ser
   245 250 255
   Ala Ala Ala Gly Leu Lys Lys Val Thr Leu Glu Leu Gly Gly Lys Ser
   260 265 270
   Pro Asn Ile Val Phe Ala Asp Ala Glu Leu Lys Lys Ala Val Gln Asn
   275 280 285
   Ile Ile Leu Gly Ile Tyr Tyr Asn Ser Gly Glu Val Cys Cys Ala Gly
   290 295 300
   Ser Arg Val Tyr Val Glu Glu Ser Ile Tyr Asp Lys Phe Ile Glu Glu
   305 310 315 320
   Phe Lys Ala Ala Ser Glu Ser Ile Lys Val Gly Asp Pro Phe Asp Glu
   325 330 335
   Ser Thr Phe Gln Gly Ala Gln Thr Ser Gln Met Gln Leu Asn Lys Ile
   340 345 350
   Leu Lys Tyr Val Asp Ile Gly Lys Asn Glu Gly Ala Thr Leu Ile Thr
   355 360 365
   Gly Gly Glu Arg Leu Gly Ser Lys Gly Tyr Phe Ile Lys Pro Thr Val
   370 375 380
   Phe Gly Asp Val Lys Glu Asp Met Arg Ile Val Lys Glu Glu Ile Phe
   385 390 395 400
   Gly Pro Val Val Thr Val Thr Lys Phe Lys Ser Ala Asp Glu Val Ile
   405 410 415
   Asn Met Ala Asn Asp Ser Glu Tyr Gly Leu Ala Ala Gly Ile His Thr
   420 425 430
   Ser Asn Ile Asn Thr Ala Leu Lys Val Ala Asp Arg Val Asn Ala Gly
   435 440 445
   Thr Val Trp Ile Asn Thr Tyr Asn Asp Phe His His Ala Val Pro Phe
   450 455 460
   Gly Gly Phe Asn Ala Ser Gly Leu Gly Arg Glu Met Ser Val Asp Ala
   465 470 475 480
   Leu Gln Asn Tyr Leu Gln Val Lys Ala Val Arg Ala Lys Leu Asp
   485 490 495
<210> 3
   <211> 1541
   <212> DNA
   <213> Homo sapiens
<220>
   <221> CDS
   <222> (22)..(1521)
<400> 3
   gcggtaccaa ggagatatca t atg tca gcc gcc gcc acc cag gcc gtg cct    51
                           Met Ser Ala Ala Ala Thr Gln Ala Val Pro
                            1               5                  10
   gcc ccc aac cag cag ccc gag gtc ttc tgc aac cag att ttc ata aac    99
   Ala Pro Asn Gln Gln Pro Glu Val Phe Cys Asn Gln Ile Phe Ile Asn
               15                  20                  25
   aat gaa tgg cac gat gcc gtc agc agg aaa aca ttc ccc acc gtc aat   147
   Asn Glu Trp His Asp Ala Val Ser Arg Lys Thr Phe Pro Thr Val Asn
           30                  35                  40
   ccg tcc act gga gag gtc atc tgt cag gta gct gaa ggg gac aag gaa   195
   Pro Ser Thr Gly Glu Val Ile Cys Gln Val Ala Glu Gly Asp Lys Glu
       45                  50                  55
   gat gtg gac aag gca cgt gaa ggc cgc ccg ggc gcc ttc cag ctg ggc   243
   Asp Val Asp Lys Ala Arg Glu Gly Arg Pro Gly Ala Phe Gln Leu Gly
   60                  65                  70
   tca cct tgg cgc cgc atg gac gca tca cac agc ggc cgg ctg ctg aac   291
   Ser Pro Trp Arg Arg Met Asp Ala Ser His Ser Gly Arg Leu Leu Asn
   75                  80                  85                  90
   cgc ctg gcc gat ctg atc gag cgg gac cgg acc tac ctg gcg gcc ttg   339
   Arg Leu Ala Asp Leu Ile Glu Arg Asp Arg Thr Tyr Leu Ala Ala Leu
                   95                  100                 105
   gag acc ctg gac aat ggc aag ccc tat gtc atc tcc tac ctg gtg gat   387
   Glu Thr Leu Asp Asn Gly Lys Pro Tyr Val Ile Ser Tyr Leu Val Asp
               110                 115                 120
   ttg gac atg gtc ctc aaa tgt ctc cgg tat tat gcc ggc tgg gct gat    435
   Leu Asp Met Val Leu Lys Cys Leu Arg Tyr Tyr Ala Gly Trp Ala Asp
           125                 130                 135
   aag tac cac ggg aaa acc atc ccc att gac gga gac ttc ttc agc tac    483
   Lys Tyr His Gly Lys Thr Ile Pro Ile Asp Gly Asp Phe Phe Ser Tyr
       140                 145                 150
   aca cgc cat gaa cct gtg ggg gtg tgc ggg cag atc att ccg tgg aat    531
   Thr Arg His Glu Pro Val Gly Val Cys Gly Gln Ile Ile Pro Trp Asn
   155                 160                 165                 170
   ttc ccg ctc ctg atg caa gca tgg aag ctg ggc cca gcc ttg gca act    579
   Phe Pro Leu Leu Met Gln Ala Trp Lys Leu Gly Pro Ala Leu Ala Thr
                   175                 180                 185
   gga aac gtg gtt gtg atg aag gta gct gag cag aca ccc ctc acc gcc    627
   Gly Asn Val Val Val Met Lys Val Ala Glu Gln Thr Pro Leu Thr Ala
               190                 195                 200
   ctc tat gtg gcc aac ctg atc aag gag gct ggc ttt ccc cct ggt gtg    675
   Leu Tyr Val Ala Asn Leu Ile Lys Glu Ala Gly Phe Pro Pro Gly Val
           205                 210                 215
   gtc aac att gtg cct gga ttt ggc ccc acg gct ggg gcc gcc att gcc    723
   Val Asn Ile Val Pro Gly Phe Gly Pro Thr Ala Gly Ala Ala Ile Ala
       220                 225                 230
   tcc cat gag gat gtg gac aaa gtg gca ttc aca ggc tcc act gag att    771
   Ser His Glu Asp Val Asp Lys Val Ala Phe Thr Gly Ser Thr Glu Ile
   235                 240                 245                 250
   ggc cgc gta atc cag gtt gct gct ggg agc agc aac ctc aag aga gtg    819
   Gly Arg Val Ile Gln Val Ala Ala Gly Ser Ser Asn Leu Lys Arg Val
                   255                 260                 265
   acc ttg gag ctg ggg ggg aag agc ccc aac atc atc atg tca gat gcc    867
   Thr Leu Glu Leu Gly Gly Lys Ser Pro Asn Ile Ile Met Ser Asp Ala
               270                 275                 280
   gat atg gat tgg gcc gtg gaa cag gcc cac ttc gcc ctg ttc ttc aac    915
   Asp Met Asp Trp Ala Val Glu Gln Ala His Phe Ala Leu Phe Phe Asn
           285                 290                 295
   cag ggc cag tgc tgc tgt gcc ggc tcc cgg acc ttc gtg cag gag gac    963
   Gln Gly Gln Cys Cys Cys Ala Gly Ser Arg Thr Phe Val Gln Glu Asp
       300                 305                 310
   atc tat gat gag ttt gtg gtg cgg agc gtt gcc cgg gcc aag tct cgg   1011
   Ile Tyr Asp Glu Phe Val Val Arg Ser Val Ala Arg Ala Lys Ser Arg
   315                 320                 325                 330
   gtg gtc ggg aac ccc ttt gat agc aag acc gag cag ggg ccg cag gtg   1059
   Val Val Gly Asn Pro Phe Asp Ser Lys Thr Glu Gln Gly Pro Gln Val
                   335                 340                 345
   gat gaa act cag ttt aag aag atc ctc ggc tac atc aac acg ggg aag   1107
   Asp Glu Thr Gln Phe Lys Lys Ile Leu Gly Tyr Ile Asn Thr Gly Lys
               350                 355                 360
   caa gag ggg gcg aag ctg ctg tgt ggt ggg ggc att gct gct gac cgt   1155
   Gln Glu Gly Ala Lys Leu Leu Cys Gly Gly Gly Ile Ala Ala Asp Arg
           365                 370                 375
   ggt tac ttc atc cag ccc act gtg ttt gga gat gtg cag gat ggc atg   1203
   Gly Tyr Phe Ile Gln Pro Thr Val Phe Gly Asp Val Gln Asp Gly Met
       380                 385                 390
   acc atc gcc aag gag gag atc ttc ggg cca gtg atg cag atc ctg aag   1251
   Thr Ile Ala Lys Glu Glu Ile Phe Gly Pro Val Met Gln Ile Leu Lys
   395                 400                 405                 410
   ttc aag acc ata gag gag gtt gtt ggg aga gcc aac aat tcc acg tac 1299
   Phe Lys Thr Ile Glu Glu Val Val Gly Arg Ala Asn Asn Ser Thr Tyr
   415 420 425
   ggg ctg gcc gca gct gtc ttc aca aag gat ttg gac aag gcc aat tac 1347
   Gly Leu Ala Ala Ala Val Phe Thr Lys Asp Leu Asp Lys Ala Asn Tyr
   430 435 440
   ctg tcc cag gcc ctc cag gcg ggc act gtg tgg gtc aac tgc tat gat 1395
   Leu Ser Gln Ala Leu Gln Ala Gly Thr Val Trp Val Asn Cys Tyr Asp
   445 450 455
   gtg ttt gga gcc cag tca ccc ttt ggt ggc tac aag atg tcg ggg agt 1443
   Val Phe Gly Ala Gln Ser Pro Phe Gly Gly Tyr Lys Met Ser Gly Ser
   460 465 470
   ggc cgg gag ttg ggc gag tac ggg ctg cag gca tac act gaa gtg aaa 1491
   Gly Arg Glu Leu Gly Glu Tyr Gly Leu Gln Ala Tyr Thr Glu Val Lys
   475 480 485 490
   act gtc aca gtc aaa gtg cct cag aag aac tcataagagc tcgaattcgc 1541
   Thr Val Thr Val Lys Val Pro Gln Lys Asn
   495 500
<210> 4
   <211> 500
   <212> PRT
   <213> Homo sapiens
<400> 4
   Met Ser Ala Ala Ala Thr Gln Ala Val Pro Ala Pro Asn Gln Gln Pro
   1 5 10 15
   Glu Val Phe Cys Asn Gln Ile Phe Ile Asn Asn Glu Trp His Asp Ala
   20 25 30
   Val Ser Arg Lys Thr Phe Pro Thr Val Asn Pro Ser Thr Gly Glu Val
   35 40 45
   Ile Cys Gln Val Ala Glu Gly Asp Lys Glu Asp Val Asp Lys Ala Arg
   50 55 60
   Glu Gly Arg Pro Gly Ala Phe Gln Leu Gly Ser Pro Trp Arg Arg Met
   65 70 75 80
   Asp Ala Ser His Ser Gly Arg Leu Leu Asn Arg Leu Ala Asp Leu Ile
   85 90 95
   Glu Arg Asp Arg Thr Tyr Leu Ala Ala Leu Glu Thr Leu Asp Asn Gly
   100 105 110
   Lys Pro Tyr Val Ile Ser Tyr Leu Val Asp Leu Asp Met Val Leu Lys
   115 120 125
   Cys Leu Arg Tyr Tyr Ala Gly Trp Ala Asp Lys Tyr His Gly Lys Thr
   130 135 140
   Ile Pro Ile Asp Gly Asp Phe Phe Ser Tyr Thr Arg His Glu Pro Val
   145 150 155 160
   Gly Val Cys Gly Gln Ile Ile Pro Trp Asn Phe Pro Leu Leu Met Gln
   165 170 175
   Ala Trp Lys Leu Gly Pro Ala Leu Ala Thr Gly Asn Val Val Val Met
   180 185 190
   Lys Val Ala Glu Gln Thr Pro Leu Thr Ala Leu Tyr Val Ala Asn Leu
   195 200 205
   Ile Lys Glu Ala Gly Phe Pro Pro Gly Val Val Asn Ile Val Pro Gly
   210 215 220
   Phe Gly Pro Thr Ala Gly Ala Ala Ile Ala Ser His Glu Asp Val Asp
   225 230 235 240
   Lys Val Ala Phe Thr Gly Ser Thr Glu Ile Gly Arg Val Ile Gln Val
   245 250 255
   Ala Ala Gly Ser Ser Asn Leu Lys Arg Val Thr Leu Glu Leu Gly Gly
   260 265 270
   Lys Ser Pro Asn Ile Ile Met Ser Asp Ala Asp Met Asp Trp Ala Val
   275 280 285
   Glu Gln Ala His Phe Ala Leu Phe Phe Asn Gln Gly Gln Cys Cys Cys
   290 295 300
   Ala Gly Ser Arg Thr Phe Val Gln Glu Asp Ile Tyr Asp Glu Phe Val
   305 310 315 320
   Val Arg Ser Val Ala Arg Ala Lys Ser Arg Val Val Gly Asn Pro Phe
   325 330 335
   Asp Ser Lys Thr Glu Gln Gly Pro Gln Val Asp Glu Thr Gln Phe Lys
   340 345 350
   Lys Ile Leu Gly Tyr Ile Asn Thr Gly Lys Gln Glu Gly Ala Lys Leu
   355 360 365
   Leu Cys Gly Gly Gly Ile Ala Ala Asp Arg Gly Tyr Phe Ile Gln Pro
   370 375 380
   Thr Val Phe Gly Asp Val Gln Asp Gly Met Thr Ile Ala Lys Glu Glu
   385 390 395 400
   Ile Phe Gly Pro Val Met Gln Ile Leu Lys Phe Lys Thr Ile Glu Glu
   405 410 415
   Val Val Gly Arg Ala Asn Asn Ser Thr Tyr Gly Leu Ala Ala Ala Val
   420 425 430
   Phe Thr Lys Asp Leu Asp Lys Ala Asn Tyr Leu Ser Gln Ala Leu Gln
   435 440 445
   Ala Gly Thr Val Trp Val Asn Cys Tyr Asp Val Phe Gly Ala Gln Ser
   450 455 460
   Pro Phe Gly Gly Tyr Lys Met Ser Gly Ser Gly Arg Glu Leu Gly Glu
   465 470 475 480
   Tyr Gly Leu Gln Ala Tyr Thr Glu Val Lys Thr Val Thr Val Lys Val
   485 490 495
   Pro Gln Lys Asn
   500
<210> 5
   <211> 1513
   <212> DNA
   <213> Escherichia coli
<220>
   <221> CDS
   <222> (37)..(1473)
<400> 5
   gctaccatgg cttaaccggt accaaggaga tatcat atg tca gta ccc gtt caa 54
   Met Ser Val Pro Val Gln
   1 5
   cat cct atg tat atc gat gga cag ttt gtt acc tgg cgt gga gac gca 102
   His Pro Met Tyr Ile Asp Gly Gln Phe Val Thr Trp Arg Gly Asp Ala
   10 15 20
   tgg att gat gtg gta aac cct gct aca gag gct gtc att tcc cgc ata   150
   Trp Ile Asp Val Val Asn Pro Ala Thr Glu Ala Val Ile Ser Arg Ile
           25                  30                  35
   ccc gat ggt cag gcc gag gat gcc cgt aag gca atc gat gca gca gaa   198
   Pro Asp Gly Gln Ala Glu Asp Ala Arg Lys Ala Ile Asp Ala Ala Glu
       40                  45                  50
   cgt gca caa cca gaa tgg gaa gcg ttg cct gct att gaa cgc gcc agt   246
   Arg Ala Gln Pro Glu Trp Glu Ala Leu Pro Ala Ile Glu Arg Ala Ser
    55                  60                  65                  70
   tgg ttg cgc aaa atc tcc gcc ggg atc cgc gaa cgc gcc agt gaa atc   294
   Trp Leu Arg Lys Ile Ser Ala Gly Ile Arg Glu Arg Ala Ser Glu Ile
                    75                  80                  85
   agt gcg ctg att gtt gaa gaa ggg ggc aag atc cag cag ctg gct gaa   342
   Ser Ala Leu Ile Val Glu Glu Gly Gly Lys Ile Gln Gln Leu Ala Glu
                90                  95                 100
   gtc gaa gtg gct ttt act gcc gac tat atc gat tac atg gcg gag tgg   390
   Val Glu Val Ala Phe Thr Ala Asp Tyr Ile Asp Tyr Met Ala Glu Trp
           105                 110                 115
   gca cgg cgt tac gag ggc gag att att caa agc gat cgt cca gga gaa   438
   Ala Arg Arg Tyr Glu Gly Glu Ile Ile Gln Ser Asp Arg Pro Gly Glu
       120                 125                 130
   aat att ctt ttg ttt aaa cgt gcg ctt ggt gtg act acc ggc att ctg   486
   Asn Ile Leu Leu Phe Lys Arg Ala Leu Gly Val Thr Thr Gly Ile Leu
   135                 140                 145                 150
   ccg tgg aac ttc ccg ttc ttc ctc att gcc cgc aaa atg gct ccc gct   534
   Pro Trp Asn Phe Pro Phe Phe Leu Ile Ala Arg Lys Met Ala Pro Ala
                   155                 160                 165
   ctt ttg acc ggt aat acc atc gtc att aaa cct agt gaa ttt acg aca   582
   Leu Leu Thr Gly Asn Thr Ile Val Ile Lys Pro Ser Glu Phe Thr Thr
               170                 175                 180
   aac aat gcg att gca ttc gcc aaa atc gtc gat gaa ata ggc ctt ccg   630
   Asn Asn Ala Ile Ala Phe Ala Lys Ile Val Asp Glu Ile Gly Leu Pro
           185                 190                 195
   cgc ggc gtg ttt aac ctt gta ctg ggg cgt ggt gaa acc gtt ggg caa   678
   Arg Gly Val Phe Asn Leu Val Leu Gly Arg Gly Glu Thr Val Gly Gln
       200                 205                 210
   gaa ctg gcg ggt aac cca aag gtc gca atg gtc agt atg aca ggc agc   726
   Glu Leu Ala Gly Asn Pro Lys Val Ala Met Val Ser Met Thr Gly Ser
   215                 220                 225                 230
   gtc tct gca ggt gag aag atc atg gcg act gcg gcg aaa aac atc acc   774
   Val Ser Ala Gly Glu Lys Ile Met Ala Thr Ala Ala Lys Asn Ile Thr
                   235                 240                 245
   aaa gtg tgt ctg gaa ttg ggg ggt aaa gca cca gct atc gta atg gac   822
   Lys Val Cys Leu Glu Leu Gly Gly Lys Ala Pro Ala Ile Val Met Asp
               250                 255                 260
   gat gcc gat ctt gaa ctg gca gtc aaa gcc atc gtt gat tca cgc gtc   870
   Asp Ala Asp Leu Glu Leu Ala Val Lys Ala Ile Val Asp Ser Arg Val
           265                 270                 275
   att aat agt ggg caa gtg tgt aac tgt gca gaa cgt gtt tat gta cag   918
   Ile Asn Ser Gly Gln Val Cys Asn Cys Ala Glu Arg Val Tyr Val Gln
       280                 285                 290
   aaa ggc att tat gat cag ttc gtc aat cgg ctg ggt gaa gcg atg cag   966
   Lys Gly Ile Tyr Asp Gln Phe Val Asn Arg Leu Gly Glu Ala Met Gln
   295                 300                 305                 310
   gcg gtt caa ttt ggt aac ccc gct gaa cgc aac gac att gcg atg ggg   1014
   Ala Val Gln Phe Gly Asn Pro Ala Glu Arg Asn Asp Ile Ala Met Gly
                   315                 320                 325
   ccg ttg att aac gcc gcg gcg ctg gaa agg gtc gag caa aaa gtg gcg   1062
   Pro Leu Ile Asn Ala Ala Ala Leu Glu Arg Val Glu Gln Lys Val Ala
               330                 335                 340
   cgc gca gta gaa gaa ggg gcg aga gtg gcg ttc ggt ggc aaa gcg gta   1110
   Arg Ala Val Glu Glu Gly Ala Arg Val Ala Phe Gly Gly Lys Ala Val
           345                 350                 355
   gag ggg aaa gga tat tat tat ccg ccg aca ttg ctg ctg gat gtt cgc   1158
   Glu Gly Lys Gly Tyr Tyr Tyr Pro Pro Thr Leu Leu Leu Asp Val Arg
       360                 365                 370
   cag gaa atg tcg att atg cat gag gaa acc ttt ggc ccg gtg ctg cca   1206
   Gln Glu Met Ser Ile Met His Glu Glu Thr Phe Gly Pro Val Leu Pro
   375                 380                 385                 390
   gtt gtc gca ttt gac acg ctg gaa gat gct atc tca atg gct aat gac   1254
   Val Val Ala Phe Asp Thr Leu Glu Asp Ala Ile Ser Met Ala Asn Asp
                   395                 400                 405
   agt gat tac ggc ctg acc tca tca atc tat acc caa aat ctg aac gtc   1302
   Ser Asp Tyr Gly Leu Thr Ser Ser Ile Tyr Thr Gln Asn Leu Asn Val
               410                 415                 420
   gcg atg aaa gcc att aaa ggg ctg aag ttt ggt gaa act tac atc aac   1350
   Ala Met Lys Ala Ile Lys Gly Leu Lys Phe Gly Glu Thr Tyr Ile Asn
           425                 430                 435
   cgt gaa aac ttc gaa gct atg caa ggc ttc cac gcc gga tgg cgt aaa   1398
   Arg Glu Asn Phe Glu Ala Met Gln Gly Phe His Ala Gly Trp Arg Lys
       440                 445                 450
   tcc ggt att ggc ggc gca gat ggt aaa cat ggc ttg cat gga tat ctg     1446
   Ser Gly Ile Gly Gly Ala Asp Gly Lys His Gly Leu His Gly Tyr Leu
   455                 460                 465                 470
   cag acc cag gtg gtt tat tta cag tct taagagctcg aattcccgtc           1493
   Gln Thr Gln Val Val Tyr Leu Gln Ser
                   475
   gacggctcta gactcgagcg                                               1513
<210> 6
   <211> 479
   <212> PRT
   <213> Escherichia coli
<400> 6
   Met Ser Val Pro Val Gln His Pro Met Tyr Ile Asp Gly Gln Phe Val
   1               5                   10                  15
   Thr Trp Arg Gly Asp Ala Trp Ile Asp Val Val Asn Pro Ala Thr Glu
               20                  25                  30
   Ala Val Ile Ser Arg Ile Pro Asp Gly Gln Ala Glu Asp Ala Arg Lys
           35                  40                  45
   Ala Ile Asp Ala Ala Glu Arg Ala Gln Pro Glu Trp Glu Ala Leu Pro
       50                  55                  60
   Ala Ile Glu Arg Ala Ser Trp Leu Arg Lys Ile Ser Ala Gly Ile Arg
   65                  70                  75                  80
   Glu Arg Ala Ser Glu Ile Ser Ala Leu Ile Val Glu Glu Gly Gly Lys
                   85                  90                  95
   Ile Gln Gln Leu Ala Glu Val Glu Val Ala Phe Thr Ala Asp Tyr Ile
               100                 105                 110
   Asp Tyr Met Ala Glu Trp Ala Arg Arg Tyr Glu Gly Glu Ile Ile Gln
   115 120 125
   Ser Asp Arg Pro Gly Glu Asn Ile Leu Leu Phe Lys Arg Ala Leu Gly
   130 135 140
   Val Thr Thr Gly Ile Leu Pro Trp Asn Phe Pro Phe Phe Leu Ile Ala
   145 150 155 160
   Arg Lys Met Ala Pro Ala Leu Leu Thr Gly Asn Thr Ile Val Ile Lys
   165 170 175
   Pro Ser Glu Phe Thr Thr Asn Asn Ala Ile Ala Phe Ala Lys Ile Val
   180 185 190
   Asp Glu Ile Gly Leu Pro Arg Gly Val Phe Asn Leu Val Leu Gly Arg
   195 200 205
   Gly Glu Thr Val Gly Gln Glu Leu Ala Gly Asn Pro Lys Val Ala Met
   210 215 220
   Val Ser Met Thr Gly Ser Val Ser Ala Gly Glu Lys Ile Met Ala Thr
   225 230 235 240
   Ala Ala Lys Asn Ile Thr Lys Val Cys Leu Glu Leu Gly Gly Lys Ala
   245 250 255
   Pro Ala Ile Val Met Asp Asp Ala Asp Leu Glu Leu Ala Val Lys Ala
   260 265 270
   Ile Val Asp Ser Arg Val Ile Asn Ser Gly Gln Val Cys Asn Cys Ala
   275 280 285
   Glu Arg Val Tyr Val Gln Lys Gly Ile Tyr Asp Gln Phe Val Asn Arg
   290 295 300
   Leu Gly Glu Ala Met Gln Ala Val Gln Phe Gly Asn Pro Ala Glu Arg
   305 310 315 320
   Asn Asp Ile Ala Met Gly Pro Leu Ile Asn Ala Ala Ala Leu Glu Arg
   325 330 335
   Val Glu Gln Lys Val Ala Arg Ala Val Glu Glu Gly Ala Arg Val Ala
   340 345 350
   Phe Gly Gly Lys Ala Val Glu Gly Lys Gly Tyr Tyr Tyr Pro Pro Thr
   355 360 365
   Leu Leu Leu Asp Val Arg Gln Glu Met Ser Ile Met His Glu Glu Thr
   370 375 380
   Phe Gly Pro Val Leu Pro Val Val Ala Phe Asp Thr Leu Glu Asp Ala
   385 390 395 400
   Ile Ser Met Ala Asn Asp Ser Asp Tyr Gly Leu Thr Ser Ser Ile Tyr
   405 410 415
   Thr Gln Asn Leu Asn Val Ala Met Lys Ala Ile Lys Gly Leu Lys Phe
   420 425 430
   Gly Glu Thr Tyr Ile Asn Arg Glu Asn Phe Glu Ala Met Gln Gly Phe
   435 440 445
   His Ala Gly Trp Arg Lys Ser Gly Ile Gly Gly Ala Asp Gly Lys His
   450 455 460
   Gly Leu His Gly Tyr Leu Gln Thr Gln Val Val Tyr Leu Gln Ser
   465 470 475
<210> 7
   <211> 1574
   <212> DNA
   <213> Escherichia coli
<220>
   <221> CDS
   <222> (22)..(1557)
<400> 7
   gcggtaccaa ggaggtatca t atg acc aat aat ccc cct tca gca cag att     51
                           Met Thr Asn Asn Pro Pro Ser Ala Gln Ile
                             1               5                  10
   aag ccc ggc gag tat ggt ttc ccc ctc aag tta aaa gcc cgc tat gac     99
   Lys Pro Gly Glu Tyr Gly Phe Pro Leu Lys Leu Lys Ala Arg Tyr Asp
                   15                  20                  25
   aac ttt att ggc ggc gaa tgg gta gcc cct gcc gac ggc gag tat tac    147
   Asn Phe Ile Gly Gly Glu Trp Val Ala Pro Ala Asp Gly Glu Tyr Tyr
               30                  35                  40
   cag aat ctg acg ccg gtg acc ggg cag ctg ctg tgc gaa gtg gcg tct    195
   Gln Asn Leu Thr Pro Val Thr Gly Gln Leu Leu Cys Glu Val Ala Ser
           45                  50                  55
   tcg ggc aaa cga gac atc gat ctg gcg ctg gat gct gcg cac aaa gtg    243
   Ser Gly Lys Arg Asp Ile Asp Leu Ala Leu Asp Ala Ala His Lys Val
       60                  65                  70
   aaa gat aaa tgg gcg cac acc tcg gtg cag gat cgt gcg gcg att ctg    291
   Lys Asp Lys Trp Ala His Thr Ser Val Gln Asp Arg Ala Ala Ile Leu
   75                  80                  85                  90
   ttt aag att gcc gat cga atg gaa caa aac ctc gag ctg tta gcg aca    339
   Phe Lys Ile Ala Asp Arg Met Glu Gln Asn Leu Glu Leu Leu Ala Thr
                   95                  100                 105
   gct gaa acc tgg gat aac ggc aaa ccc att cgc gaa acc agt gct gcg    387
   Ala Glu Thr Trp Asp Asn Gly Lys Pro Ile Arg Glu Thr Ser Ala Ala
               110                 115                 120
   gat gta ccg ctg gcg att gac cat ttc cgc tat ttc gcc tcg tgt att    435
   Asp Val Pro Leu Ala Ile Asp His Phe Arg Tyr Phe Ala Ser Cys Ile
           125                 130                 135
   cgg gcg cag gaa ggt ggg atc agt gaa gtt gat agc gaa acc gtg gcc    483
   Arg Ala Gln Glu Gly Gly Ile Ser Glu Val Asp Ser Glu Thr Val Ala
       140                 145                 150
   tat cat ttc cat gaa ccg tta ggc gtg gtg ggg cag att atc ccg tgg    531
   Tyr His Phe His Glu Pro Leu Gly Val Val Gly Gln Ile Ile Pro Trp
   155                 160                 165                 170
   aac ttc ccg ctg ctg atg gcg agc tgg aaa atg gct ccc gcg ctg gcg    579
   Asn Phe Pro Leu Leu Met Ala Ser Trp Lys Met Ala Pro Ala Leu Ala
                   175                 180                 185
   gcg ggc aac tgt gtg gtg ctg aaa ccc gca cgt ctt acc ccg ctt tct    627
   Ala Gly Asn Cys Val Val Leu Lys Pro Ala Arg Leu Thr Pro Leu Ser
               190                 195                 200
   gta ctg ctg cta atg gaa att gtc ggt gat tta ctg ccg ccg ggc gtg    675
   Val Leu Leu Leu Met Glu Ile Val Gly Asp Leu Leu Pro Pro Gly Val
           205                 210                 215
   gtg aac gtg gtc aat ggc gca ggt ggg gta att ggc gaa tat ctg gcg    723
   Val Asn Val Val Asn Gly Ala Gly Gly Val Ile Gly Glu Tyr Leu Ala
       220                 225                 230
   acc tcg aaa cgc atc gcc aaa gtg gcg ttt acc ggc tca acg gaa gtg    771
   Thr Ser Lys Arg Ile Ala Lys Val Ala Phe Thr Gly Ser Thr Glu Val
   235                 240                 245                 250
   ggc caa caa att atg caa tac gca acg caa aac att att ccg gtg acg   819
   Gly Gln Gln Ile Met Gln Tyr Ala Thr Gln Asn Ile Ile Pro Val Thr
                   255                 260                 265
   ctg gag ttg ggc ggt aag tcg cca aat atc gtc ttt gct gat gtg atg   867
   Leu Glu Leu Gly Gly Lys Ser Pro Asn Ile Val Phe Ala Asp Val Met
               270                 275                 280
   gat gaa gaa gat gcc ttt ttc gat aaa gcg ctg gaa ggc ttt gca ctg   915
   Asp Glu Glu Asp Ala Phe Phe Asp Lys Ala Leu Glu Gly Phe Ala Leu
           285                 290                 295
   ttt gcc ttt aac cag ggc gaa gtt tgc acc tgt ccg agt cgt gct tta   963
   Phe Ala Phe Asn Gln Gly Glu Val Cys Thr Cys Pro Ser Arg Ala Leu
       300                 305                 310
   gtg cag gaa tct atc tac gaa cgc ttt atg gaa cgc gcc atc cgc cgt   1011
   Val Gln Glu Ser Ile Tyr Glu Arg Phe Met Glu Arg Ala Ile Arg Arg
   315                 320                 325                 330
   gtc gaa agc att cgt agc ggt aac ccg ctc gac agc gtg acg caa atg   1059
   Val Glu Ser Ile Arg Ser Gly Asn Pro Leu Asp Ser Val Thr Gln Met
                   335                 340                 345
   ggc gcg cag gtt tct cac ggg caa ctg gaa acc atc ctc aac tac att   1107
   Gly Ala Gln Val Ser His Gly Gln Leu Glu Thr Ile Leu Asn Tyr Ile
               350                 355                 360
   gat atc ggt aaa aaa gag ggc gct gac gtg ctc aca ggc ggg cgg cgc   1155
   Asp Ile Gly Lys Lys Glu Gly Ala Asp Val Leu Thr Gly Gly Arg Arg
           365                 370                 375
   aag ctg ctg gaa ggt gaa ctg aaa gac ggc tac tac ctc gaa ccg acg   1203
   Lys Leu Leu Glu Gly Glu Leu Lys Asp Gly Tyr Tyr Leu Glu Pro Thr
       380                 385                 390
   att ctg ttt ggt cag aac aat atg cgg gtg ttc cag gag gag att ttt   1251
   Ile Leu Phe Gly Gln Asn Asn Met Arg Val Phe Gln Glu Glu Ile Phe
   395                 400                 405                 410
   ggc ccg gtg ctg gcg gtg acc acc ttc aaa acg atg gaa gaa gcg ctg   1299
   Gly Pro Val Leu Ala Val Thr Thr Phe Lys Thr Met Glu Glu Ala Leu
                   415                 420                 425
   gag ctg gcg aac gat acg caa tat ggc ctg ggc gcg ggc gtc tgg agc   1347
   Glu Leu Ala Asn Asp Thr Gln Tyr Gly Leu Gly Ala Gly Val Trp Ser
               430                 435                 440
   cgc aac ggt aat ctg gcc tat aag atg ggg cgc ggc ata cag gct ggg   1395
   Arg Asn Gly Asn Leu Ala Tyr Lys Met Gly Arg Gly Ile Gln Ala Gly
           445                 450                 455
   cgc gtg tgg acc aac tgt tat cac gct tac ccg gca cat gcg gcg ttt   1443
   Arg Val Trp Thr Asn Cys Tyr His Ala Tyr Pro Ala His Ala Ala Phe
       460                 465                 470
   ggt ggc tac aaa caa tca ggt atc ggt cgc gaa acc cac aag atg atg   1491
   Gly Gly Tyr Lys Gln Ser Gly Ile Gly Arg Glu Thr His Lys Met Met
   475                 480                 485                 490
   ctg gag cat tac cag caa acc aag tgc ctg ctg gtg agc tac tcg gat   1539
   Leu Glu His Tyr Gln Gln Thr Lys Cys Leu Leu Val Ser Tyr Ser Asp
                   495                 500                 505
   aaa ccg ttg ggg ctg ttc taagagctcg aattcgc                        1574
   Lys Pro Leu Gly Leu Phe
               510
<210> 8
   <211> 512
   <212> PRT
   <213> Escherichia coli
<400> 8
   Met Thr Asn Asn Pro Pro Ser Ala Gln Ile Lys Pro Gly Glu Tyr Gly
   1 5 10 15
   Phe Pro Leu Lys Leu Lys Ala Arg Tyr Asp Asn Phe Ile Gly Gly Glu
   20 25 30
   Trp Val Ala Pro Ala Asp Gly Glu Tyr Tyr Gln Asn Leu Thr Pro Val
   35 40 45
   Thr Gly Gln Leu Leu Cys Glu Val Ala Ser Ser Gly Lys Arg Asp Ile
   50 55 60
   Asp Leu Ala Leu Asp Ala Ala His Lys Val Lys Asp Lys Trp Ala His
   65 70 75 80
   Thr Ser Val Gln Asp Arg Ala Ala Ile Leu Phe Lys Ile Ala Asp Arg
   85 90 95
   Met Glu Gln Asn Leu Glu Leu Leu Ala Thr Ala Glu Thr Trp Asp Asn
   100 105 110
   Gly Lys Pro Ile Arg Glu Thr Ser Ala Ala Asp Val Pro Leu Ala Ile
   115 120 125
   Asp His Phe Arg Tyr Phe Ala Ser Cys Ile Arg Ala Gln Glu Gly Gly
   130 135 140
   Ile Ser Glu Val Asp Ser Glu Thr Val Ala Tyr His Phe His Glu Pro
   145 150 155 160
   Leu Gly Val Val Gly Gln Ile Ile Pro Trp Asn Phe Pro Leu Leu Met
   165 170 175
   Ala Ser Trp Lys Met Ala Pro Ala Leu Ala Ala Gly Asn Cys Val Val
   180 185 190
   Leu Lys Pro Ala Arg Leu Thr Pro Leu Ser Val Leu Leu Leu Met Glu
   195 200 205
   Ile Val Gly Asp Leu Leu Pro Pro Gly Val Val Asn Val Val Asn Gly
   210 215 220
   Ala Gly Gly Val Ile Gly Glu Tyr Leu Ala Thr Ser Lys Arg Ile Ala
   225 230 235 240
   Lys Val Ala Phe Thr Gly Ser Thr Glu Val Gly Gln Gln Ile Met Gln
   245 250 255
   Tyr Ala Thr Gln Asn Ile Ile Pro Val Thr Leu Glu Leu Gly Gly Lys
   260 265 270
   Ser Pro Asn Ile Val Phe Ala Asp Val Met Asp Glu Glu Asp Ala Phe
   275 280 285
   Phe Asp Lys Ala Leu Glu Gly Phe Ala Leu Phe Ala Phe Asn Gln Gly
   290 295 300
   Glu Val Cys Thr Cys Pro Ser Arg Ala Leu Val Gln Glu Ser Ile Tyr
   305 310 315 320
   Glu Arg Phe Met Glu Arg Ala Ile Arg Arg Val Glu Ser Ile Arg Ser
   325 330 335
   Gly Asn Pro Leu Asp Ser Val Thr Gln Met Gly Ala Gln Val Ser His
   340 345 350
   Gly Gln Leu Glu Thr Ile Leu Asn Tyr Ile Asp Ile Gly Lys Lys Glu
   355 360 365
   Gly Ala Asp Val Leu Thr Gly Gly Arg Arg Lys Leu Leu Glu Gly Glu
   370 375 380
   Leu Lys Asp Gly Tyr Tyr Leu Glu Pro Thr Ile Leu Phe Gly Gln Asn
   385 390 395 400
   Asn Met Arg Val Phe Gln Glu Glu Ile Phe Gly Pro Val Leu Ala Val
   405 410 415
   Thr Thr Phe Lys Thr Met Glu Glu Ala Leu Glu Leu Ala Asn Asp Thr
   420 425 430
   Gln Tyr Gly Leu Gly Ala Gly Val Trp Ser Arg Asn Gly Asn Leu Ala
   435 440 445
   Tyr Lys Met Gly Arg Gly Ile Gln Ala Gly Arg Val Trp Thr Asn Cys
   450 455 460
   Tyr His Ala Tyr Pro Ala His Ala Ala Phe Gly Gly Tyr Lys Gln Ser
   465 470 475 480
   Gly Ile Gly Arg Glu Thr His Lys Met Met Leu Glu His Tyr Gln Gln
   485 490 495
   Thr Lys Cys Leu Leu Val Ser Tyr Ser Asp Lys Pro Leu Gly Leu Phe
   500 505 510
<210> 9
   <211> 5268
   <212> DNA
   <213> Klebsiella pneumoniae
<220>
   <223> Location complement 300..2153
<220>
   <223> Location complement 2166...2591
<220>
   <223> Locaton complement 2594..3034
<220>
   <223> Location complement 2191..4858
<400> 9
   agcgctatat gcgttgatgc aatttctatg cgcacccgtt ctcggagcac tgtccgaccg 60
   ctttggccgc cgcccagtcc tgctcgcttc gctacttgga gccactatcg actacgcgat 120
   catggcgacc acacccgtcc tgtggatctc ccactgacca aagctggccc cggcgacccg 180
   cagcgcagcg acgcagcccg cgccgaagaa aatgagcaat ccggtgccaa gaaactcggc 240
   cacgcactgc ccggttaagg tagaagtctg gttcattatc ggcatcctga aatagcacgt 300
   taaagagaga ggctggcgcg agcgcccgtt taattcgcct gaccggccag tagcagcccg 360
   gtggcgaccg cattgcgcgg cccttctgtt ccccgaatat tgccctgccc ggcgaccacg 420
   ccatagtgcg acaaggcttc cgtgataagc tgcgggatct caaagtccag cgatgagccg 480
   cccaccagca ccacaaaggc gatatcgcga atggaaccgc cgggtgagac ctggcgcagc 540
   gcgcgcaggc agttggtgac aaacactttc tctttcgcct gccggcgcac gagacgaatt 600
   ttttccagcg ggctggcgtt atcgatcggc accagttcgc cctccttgat gtacaccact 660
   ttggcgaaca ccgccgggct gagggcttcc cgaaagaact ccaccgcgcc attctcgtga 720
   cgaatactga acaggctttc cactttggcc agcgggtatt tttttatcgc ttccgccagc 780
   gaaagatcct cgaggcccag ctcggtttta atcaacaggc tgaccatatt ccccgccccg 840
   gcgagatgga ccgccgttat ctgcccctcc gcgttgacga tcgccgcatc cgtcgagccg 900
   gcgccgaggt cgaggatcgc cagcggcgcc gcacagccgg gagtggttaa cgccccggcg 960
   atggccatgt tggcctccac gccgcccacc accacctcgg tctgcagtcg ggcgctcagt 1020
   tcgcgggcga taacctgcat ttgcagacga tccgctttca ccatcgccgc catcccgacg 1080
   gcattctcca tggcgcactc gccggccatc ccgccctgca ccttgcgcgg aataaacgta 1140
   tccaccgcca gcagatcctg gatgtatatc gcgctcatct catggccggt cagggacgcc 1200
   attaccttgc gcacccgctc aagcatgccg ccggcgtggg tgcccggttc gccgcggatg 1260
   tcgcgtaccg gagcgcaggc gctcatcgcc tgcatgatgg cttccgcgcc ctcggcgaca 1320
   tcggcctctc cgcggcgctt ttcgccgcta atgtagaggt tgcccgccgg gatcacccgc 1380
   gactgcacat ccccctgcgg ggtcttgagc accaccgcgg aacggttgcc aatcagggcg 1440
   cgggcgatgg ggacgatggc ctgggtctct tccgggctta gcccgaagaa ggtggcgatc 1500
   ccgtagggat tcgacaggat ccgcaccacc tggcccggcg cggccacttc caccgccgcc 1560
   attaccccct cggggacctg ctccagcagc gtcacttcat ccaccaccgg cagggtttta 1620
   cgcaggcggt tgttcaccag cacgccgtcg tcctttttga ggatcgccgc caccacgttg 1680
   atcccccggt cgagcgcctc attgagccac cacacggcgt caaggaaatc gacggcgtcg 1740
   tcaatcagta cgatccaccc ctcggcatac tgcgccgccg gcagcgtcgc cagccgcccg 1800
   agggcgatag tcgtccccac gccaacgccc accccgcccg gcgtctgcgg gttatgaccg 1860
   atcatggtcg attcggtgat aatggtctcg gtgatggtct ccatcgccac atcgccaatc 1920
   accggcgcgg cttcgttaag atagatgcga gagacatcgc tcatcgacca cggtgttttc 1980
   gccagggcct gctccagcgc ggcgagggtc ccggcgatat tgtcccgcgt ccctttcatg 2040
   cccgtcgtcg cgacgatccc gctggcaaca aacgccctcg cctgcgggta gtcggacgcc 2100
   agcgccacct cggtggtggc gttgccgata tcaatcccgg ctattaacgg catgctgacc 2160
   tccgcttagc ttcctttacg cagcttatgc cgctgctgat acacttccgc cgactcccgg 2220
   acaaaggcgg cattcactgt cgcatgccag gtgtgctcca gctcgtcggc gatcgccagc 2280
   agctccgcct gcgaggagcg gaacgggcgc agcgcgttat agatagccag aatgcgctcg 2340
   tcaggaatgg cgataagctc cgccgcgcgg cggaaattgc gcgccaccgc atggcgctgc 2400
   atctgctcgg caatctgcgc ctggtactca agggtctggc gggagatccg cacatcctgc 2460
   gggcccacct cgccagagag caccttctcg agggtaatat cggtcaatgg tttgccggta 2520
   ggcgtcagga tatgctccgg gcagcgggtg gctaacggat aatcctgcac gcgcatggtt 2580
   ttctcgctca tggtcactcc cttactaagt cgatgtgcag ggtgacgggc tcggcgtcct 2640
   gcaccacatg tttggtctct ttgatatgaa atagcgcggc tttggccata aatttcggcc 2700
   gcaccatctg atcgttcacc accggcaccg gcgaaggtga ctctttgcgc gcatagcgcg 2760
   cagcgttttt gccaatctgc cggtaggtct ccagcgtcag cagcggcgcc tgggagaaca 2820
   gctccaggtt gctgagcggc agcagatcgc gctgatggat gaccgtggtc cccttcgact 2880
   ggataccgat gccgatcccc gagccgctca ggttggccgc atcccaggcc ataaaggaga 2940
   cgtcggacgt gcgcagaatg cgcaccaccc gggcgtgaag cccctcttct tccaccccgg 3000
   caatcagctc tttgaggatc gcgccatggg gcatatcgat cagagtgtga tgctggtgtt 3060
   tatcgaaggc agggccgacg ccgatcacca cttcatcggc gcgttcatcg gcagaagcta 3120
   ccccgccctc gcgggttttc agggtaaaag agggctgaat ttgggttgtc tgttgcacag 3180
   gaataccgcc ttattcaatg gtgtcgggct gaaccacgcc cggaatattt ttgatctccg 3240
   cccagcgttc ggcagagatg cgatagccgg tgcccggccc ctgatagtca ttgatgtcgt 3300
   tgaccgcact caccacctcg aactgccgat cgagaatggc cgaggtctgc aggtaatcgc 3360
   cggtgacccg ctggcgcagc atattgagaa tattgctggc gatatcctca aagccgctgc 3420
   ggctcagcgc gccgacaata tcgaggccgg tgatgttgcg cttcatcatc tcttccaccg 3480
   cactcagatc ctccaccacg ttacgcggcg gcatctcgtt gctgccgtgc gcgtaggtgg 3540
   cggcctccac ctcctcgtcg gcgattggcg gcagccccag ctcgcggaaa accgcctgga 3600
   tcgcccgcgc cgctttctgg cgaatggcaa tggtttccgc ctcggtcacc ggacgcaggc 3660
   cgccgtcaac catcaggtca cgctgcagga tgttgtaatc atcaaaatct tccgcatcga 3720
   agttcgagcc ggcgaacatg ttgtcgtagt tcggcaccgc gctgtagccg gagaaaataa 3780
   agtcggtgcc cggcagcatc tgcatcaggg tgcgcgcggt gcggcgaata tccgagtggg 3840
   agaaagtctg gtcgttggcg gacgccactt cgaggtcgag catagaggcg atcaggtttt 3900
   ccgccagcac cgcccgaatg cccgacggca cagcgccggt catgccgata cagctcaccg 3960
   cgccgttttg cagtccctga accccggcgc ctttagtaat gaagatgcag cgcgattcga 4020
   ggtagagcat cgacttgctc tccgaatagc ccatcagcgc ttcggatccg gtgccggagg 4080
   tgtagcgcat tttcaacccg cgggaggcgt aggccgaggc gaggaacgcc tttgaccacg 4140
   gcgtatcatc gccgtcggta aataccgctt cggtgccgta gaccgacacc gtctcggcgt 4200
   agctggttaa gccacgcatg cccagctcca gctcggtggc ctcttccacc gagcactgcg 4260
   tcaacacgcc ggggcggccg cactgcgaac cgaccaacag cgccagggcg ttaaacggcg 4320
   cgtagcgcgc gataccgacc gtggtctcct gttctgagaa gccgcggatc ccggcctcgg 4380
   cggcgtcagc ggcaatctgc accggattat ctttgagatt ggtgacgtgg cactggttgg 4440
   agggggtccg gcgggcacgc atcttctgca gcgccatcat catctccacc acgttcatct 4500
   gcgccatcac ctcgaccgct ttggccggcg tgatggcggt agtgatggca atgatctcct 4560
   cccggctgac gtgaatatcc accagcatac gggctatttc caccgcctcc aggcgcattg 4620
   cctgctctgt gcgctcaacg ttgatcgcgt aatcggcgat aaatcggtcg atcatgtcaa 4680
   actggtcccg gcgtttgccg tccagttcga cgatcagacc gttgtccact tttactgaag 4740
   agaccgggtc aaaggggctg tccatggcga tcagcccctc ttcaggccac tcgccaatca 4800
   gcccgtcctg attgacgggg cgctgggcca gtactgcaaa tcgttttgat cttttcattg 4860
   ttcatcggct caaaaggtga aatccgcaga cggtagcgaa tacgccgggc cagcgtcgtt 4920
   gccgcccggc cattaccggc aatagcggaa ctttaaatga gccagtggtg aaaaaaataa 4980
   atttaatttc gtttcaattt ggcacacgaa atctaccgac agtttcacta tgaaacttta 5040
   ctccggcggc aaaaataaaa aatgtgatcg cccgcaatga tataaatcaa ttaataaaaa 5100
   acgcccttaa ttacgttttt ccgacgctat tttaacccta ttgactaaat catggcgggc 5160
   gacaaaataa cgctgacaaa aataaagcaa gccaaccgaa tggtaatagt tttttactat 5220
   cgccccctac tgactattcg cgccagcgtt atcctggtgc gggagaga 5268
<210> 10
   <211> 607
   <212> PRT
   <213> Klebsiella pneumoniae
<400> 10
   Met Pro Leu Ile Ala Gly Ile Asp Ile Gly Asn Ala Thr Thr Glu Val
   1               5                   10                  15
   Ala Leu Ala Ser Asp Tyr Pro Gln Ala Arg Ala Phe Val Ala Ser Gly
   20 25 30
   Ile Val Ala Thr Thr Gly Met Lys Gly Thr Arg Asp Asn Ile Ala Gly
   35 40 45
   Thr Leu Ala Ala Leu Glu Gln Ala Leu Ala Lys Thr Pro Trp Ser Met
   50 55 60
   Ser Asp Val Ser Arg Ile Tyr Leu Asn Glu Ala Ala Pro Val Ile Gly
   65 70 75 80
   Asp Val Ala Met Glu Thr Ile Thr Glu Thr Ile Ile Thr Glu Ser Thr
   85 90 95
   Met Ile Gly His Asn Pro Gln Thr Pro Gly Gly Val Gly Val Gly Val
   100 105 110
   Gly Thr Thr Ile Ala Leu Gly Arg Leu Ala Thr Leu Pro Ala Ala Gln
   115 120 125
   Tyr Ala Glu Gly Trp Ile Val Leu Ile Asp Asp Ala Val Asp Phe Leu
   130 135 140
   Asp Ala Val Trp Trp Leu Asn Glu Ala Leu Asp Arg Gly Ile Asn Val
   145 150 155 160
   Val Ala Ala Ile Leu Lys Lys Asp Asp Gly Val Leu Val Asn Asn Arg
   165 170 175
   Leu Arg Lys Thr Leu Pro Val Val Asp Glu Val Thr Leu Leu Glu Gln
   180 185 190
   Val Pro Glu Gly Val Met Ala Ala Val Glu Val Ala Ala Pro Gly Gln
   195 200 205
   Val Val Arg Ile Leu Ser Asn Pro Tyr Gly Ile Ala Thr Phe Phe Gly
       210                 215                 220
   Leu Ser Pro Glu Glu Thr Gln Ala Ile Val Pro Ile Ala Arg Ala Leu
   225                 230                 235                 240
   Ile Gly Asn Arg Ser Ala Val Val Leu Lys Thr Pro Gln Gly Asp Val
                   245                 250                 255
   Gln Ser Arg Val Ile Pro Ala Gly Asn Leu Tyr Ile Ser Gly Glu Lys
               260                 265                 270
   Arg Arg Gly Glu Ala Asp Val Ala Glu Gly Ala Glu Ala Ile Met Gln
           275                 280                 285
   Ala Met Ser Ala Cys Ala Pro Val Arg Asp Ile Arg Gly Glu Pro Gly
       290                 295                 300
   Thr His Ala Gly Gly Met Leu Glu Arg Val Arg Lys Val Met Ala Ser
   305                 310                 315                 320
   Leu Thr Gly His Glu Met Ser Ala Ile Tyr Ile Gln Asp Leu Leu Ala
                   325                 330                 335
   Val Asp Thr Phe Ile Pro Arg Lys Val Gln Gly Gly Met Ala Gly Glu
               340                 345                 350
   Cys Ala Met Glu Asn Ala Val Gly Met Ala Ala Met Val Lys Ala Asp
           355                 360                 365
   Arg Leu Gln Met Gln Val Ile Ala Arg Glu Leu Ser Ala Arg Leu Gln
       370                 375                 380
   Thr Glu Val Val Val Gly Gly Val Glu Ala Asn Met Ala Ile Ala Gly
   385                 390                 395                 400
   Ala Leu Thr Thr Pro Gly Cys Ala Ala Pro Leu Ala Ile Leu Asp Leu
   405 410 415
   Gly Ala Gly Ser Thr Asp Ala Ala Ile Val Asn Ala Glu Gly Gln Ile
   420 425 430
   Thr Ala Val His Leu Ala Gly Ala Gly Asn Met Val Ser Leu Leu Ile
   435 440 445
   Lys Thr Glu Leu Gly Leu Glu Asp Leu Ser Leu Ala Glu Ala Ile Lys
   450 455 460
   Lys Tyr Pro Leu Ala Lys Val Glu Ser Leu Phe Ser Ile Arg His Glu
   465 470 475 480
   Asn Gly Ala Val Glu Phe Phe Arg Glu Ala Leu Ser Pro Ala Val Phe
   485 490 495
   Ala Lys Val Val Tyr Ile Lys Glu Gly Glu Leu Val Pro Ile Asp Asn
   500 505 510
   Ala Ser Pro Leu Glu Lys Ile Arg Leu Val Arg Arg Gln Ala Lys Glu
   515 520 525
   Lys Val Phe Val Thr Asn Cys Leu Arg Ala Leu Arg Gln Val Ser Pro
   530 535 540
   Gly Gly Ser Ile Arg Asp Ile Ala Phe Val Val Leu Val Gly Gly Ser
   545 550 555 560
   Ser Leu Asp Phe Glu Ile Pro Gln Leu Ile Thr Glu Ala Leu Ser His
   565 570 575
   Tyr Gly Val Val Ala Gly Gln Gly Asn Ile Arg Gly Thr Glu Gly Pro
   580 585 590
   Arg Asn Ala Val Ala Thr Gly Leu Leu Leu Ala Gly Gln Ala Asn
   595 600 605
<210> 11
   <211> 141
   <212> PRT
   <213> Klebsiella pneumoniae
<400> 11
   Met Ser Glu Lys Thr Met Arg Val Gln Asp Tyr Pro Leu Ala Thr Arg
   1               5                   10                  15
   Cys Pro Glu His Ile Leu Thr Pro Thr Gly Lys Pro Leu Thr Asp Ile
               20                  25                  30
   Thr Leu Glu Lys Val Leu Ser Gly Glu Val Gly Pro Gln Asp Val Arg
           35                  40                  45
   Ile Ser Arg Gln Thr Leu Glu Tyr Gln Ala Gln Ile Ala Glu Gln Met
       50                  55                  60
   Gln Arg His Ala Val Ala Arg Asn Phe Arg Arg Ala Ala Glu Leu Ile
   65                  70                  75                  80
   Ala Ile Pro Asp Glu Arg Ile Leu Ala Ile Tyr Asn Ala Leu Arg Pro
                   85                  90                  95
   Phe Arg Ser Ser Gln Ala Glu Leu Leu Ala Ile Ala Asp Glu Leu Glu
               100                 105                 110
   His Thr Trp His Ala Thr Val Asn Ala Ala Phe Val Arg Glu Ser Ala
           115                 120                 125
   Glu Val Tyr Gln Gln Arg His Lys Leu Arg Lys Gly Ser
       130                 135                 140
<210> 12
   <211> 146
   <212> PRT
   <213> Klebsiella pneumoniae
<400> 12
   Met Pro His Gly Ala Ile Leu Lys Glu Leu Ile Ala Gly Val Glu Glu
   1               5                   10                  15
   Glu Gly Leu His Ala Arg Val Val Arg Ile Leu Arg Thr Ser Asp Val
               20                  25                  30
   Ser Phe Met Ala Trp Asp Ala Ala Asn Leu Ser Gly Ser Gly Ile Gly
           35                  40                  45
   Ile Gly Ile Gln Ser Lys Gly Thr Thr Val Ile His Gln Arg Asp Leu
       50                  55                  60
   Leu Pro Leu Ser Asn Leu Glu Leu Phe Ser Gln Ala Pro Leu Leu Thr
   65                  70                  75                  80
   Leu Glu Thr Tyr Arg Gln Ile Gly Lys Asn Ala Ala Arg Tyr Ala Arg
                   85                  90                  95
   Lys Glu Ser Pro Ser Pro Val Pro Val Val Asn Asp Gln Met Val Arg
               100                 105                 110
   Pro Lys Phe Met Ala Lys Ala Ala Leu Phe His Ile Lys Glu Thr Lys
           115                 120                 125
   His Val Val Gln Asp Ala Glu Pro Val Thr Leu His Ile Asp Leu Val
       130                 135                 140
   Arg Glu
   145
<210> 13
   <211> 555
   <212> PRT
   <213> Klebsiella pneumoniae
<400> 13
   Met Lys Arg Ser Lys Arg Phe Ala Val Leu Ala Gln Arg Pro Val Asn
   1 5 10 15
   Gln Asp Gly Leu Ile Gly Glu Trp Pro Glu Glu Gly Leu Ile Ala Met
   20 25 30
   Asp Ser Pro Phe Asp Pro Val Ser Ser Val Lys Val Asp Asn Gly Leu
   35 40 45
   Ile Val Glu Leu Asp Gly Lys Arg Arg Asp Gln Phe Asp Met Ile Asp
   50 55 60
   Arg Phe Ile Ala Asp Tyr Ala Ile Asn Val Glu Arg Thr Glu Gln Ala
   65 70 75 80
   Met Arg Leu Glu Ala Val Glu Ile Ala Arg Met Leu Val Asp Ile His
   85 90 95
   Val Ser Arg Glu Glu Ile Ile Ala Ile Thr Thr Ala Ile Thr Pro Ala
   100 105 110
   Lys Ala Val Glu Val Met Ala Gln Met Asn Val Val Glu Met Met Met
   115 120 125
   Ala Leu Gln Lys Met Arg Ala Arg Arg Thr Pro Ser Asn Gln Cys His
   130 135 140
   Val Thr Asn Leu Lys Asp Asn Pro Val Gln Ile Ala Ala Asp Ala Ala
   145 150 155 160
   Glu Ala Gly Ile Arg Gly Phe Ser Glu Gln Glu Thr Thr Val Gly Ile
   165 170 175
   Ala Arg Tyr Ala Pro Phe Asn Ala Leu Ala Leu Leu Val Gly Ser Gln
   180 185 190
   Cys Gly Arg Pro Gly Val Leu Thr Gln Cys Ser Val Glu Glu Ala Thr
   195 200 205
   Glu Leu Glu Leu Gly Met Arg Gly Leu Thr Ser Tyr Ala Glu Thr Val
   210 215 220
   Ser Val Tyr Gly Thr Glu Ala Val Phe Thr Asp Gly Asp Asp Thr Pro
   225 230 235 240
   Trp Ser Lys Ala Phe Leu Ala Ser Ala Tyr Ala Ser Arg Gly Leu Lys
   245 250 255
   Met Arg Tyr Thr Ser Gly Thr Gly Ser Glu Ala Leu Met Gly Tyr Ser
   260 265 270
   Glu Ser Lys Ser Met Leu Tyr Leu Glu Ser Arg Cys Ile Phe Ile Thr
   275 280 285
   Lys Gly Ala Gly Val Gln Gly Leu Gln Asn Gly Ala Val Ser Cys Ile
   290 295 300
   Gly Met Thr Gly Ala Val Pro Ser Gly Ile Arg Ala Val Leu Ala Glu
   305 310 315 320
   Asn Leu Ile Ala Ser Met Leu Asp Leu Glu Val Ala Ser Ala Asn Asp
   325 330 335
   Gln Thr Phe Ser His Ser Asp Ile Arg Arg Thr Ala Arg Thr Leu Met
   340 345 350
   Gln Met Leu Pro Gly Thr Asp Phe Ile Phe Ser Gly Tyr Ser Ala Val
   355 360 365
   Pro Asn Tyr Asp Asn Met Phe Ala Gly Ser Asn Phe Asp Ala Glu Asp
   370 375 380
   Phe Asp Asp Tyr Asn Ile Leu Gln Arg Asp Leu Met Val Asp Gly Gly
   385 390 395 400
   Leu Arg Pro Val Thr Glu Ala Glu Thr Ile Ala Ile Arg Gln Lys Ala
   405 410 415
   Ala Arg Ala Ile Gln Ala Val Phe Arg Glu Leu Gly Leu Pro Pro Ile
   420 425 430
   Ala Asp Glu Glu Val Glu Ala Ala Thr Tyr Ala His Gly Ser Asn Glu
   435 440 445
   Met Pro Pro Arg Asn Val Val Glu Asp Leu Ser Ala Val Glu Glu Met
   450 455 460
   Met Lys Arg Asn Ile Thr Gly Leu Asp Ile Val Gly Ala Leu Ser Arg
   465 470 475 480
   Ser Gly Phe Glu Asp Ile Ala Ser Asn Ile Leu Asn Met Leu Arg Gln
   485 490 495
   Arg Val Thr Gly Asp Tyr Leu Gln Thr Ser Ala Ile Leu Asp Arg Gln
   500 505 510
   Phe Glu Val Val Ser Ala Val Asn Asp Ile Asn Asp Tyr Gln Gly Pro
   515 520 525
   Gly Thr Gly Tyr Arg Ile Ser Ala Glu Arg Trp Ala Glu Ile Lys Asn
   530 535 540
   Ile Pro Gly Val Val Gln Pro Asp Thr Ile Glu
   545 550 555
<210> 14
   <211> 56
   <212> DNA
   <213> Escherichia coli
<400> 14
   gctaccatgg cttaaccggt accaaggaga tatcatatgt cagtacccgt tcaaca 56
<210> 15
   <211> 59
   <212> DNA
   <212> Escherichia coli
<400> 15
   gcctcgagtc tagagccgtc gacgggaatt cgagccctta agactgtaaa taaaccacc 59
<210> 16
   <211> 46
   <212> DNA
   <213> Saccharomyces cerevisiae
<400> 16
   gcggtaccaa ggaggtatca tatgttcagt agatctacgc tctgct 46
<210> 17
   <211> 34
   <212> DNA
   <213> Saccharomyces cerevisiae
<400> 17
   gcgaattcga gctcttactc gtccaatttg gcac 34
<210> 18
   <211> 35
   <212> DNA
   <213> Homo sapiens
<400> 18
   gcggtaccaa ggaggtatca tatgtcagcc gccgc 35
<210> 19
   <211> 39
   <212> DNA
   <213> Homo sapiens
<400> 19
   gcgaattcga gctcttatga gttcttctga ggcactttg 39
<210> 20
   <211> 44
   <212> DNA
   <213> Escherichia coli
<400> 20
   gcggtaccaa ggaggtatca tatgaccaat aatccccctt cagc 44
<210> 21
   <211> 38
   <212> DNA
   <213> Escherichia coli
<400> 21
   gcgaattcga gctcttagaa cagccccaac ggtttatc 38
<210> 22
   <211> 20
   <212> DNA
   <213> Escherichia coli
<400> 22
   atcccgccgt taaccaccat . 20
<210> 23
   <211> 34
   <212> DNA
   <213> Escherichia coli
<400> 23
   gcggtaccat tgttatccgc tcacaatcc acac 34

## Description

### BACKGROUND OF THE INVENTION

The technology of genetic engineering allows the transfer of genetic traits between species and permits, in particular, the transfer of enzymes from one species to others. These techniques have first reached commercialization in connection with high-value added products such as pharmaceuticals. The techniques of genetic engineering are equally applicable and cost effective when applied to genes and enzymes which can be used to make basic chemical feedstocks.

A metabolic pathway of interest exists in the bacteria *Klebsiella pneumoniae,* which has the ability to biologically produce 3 - hydroxypropionaldehyde from glycerol. Native microorganisms have the ability to produce 1,3 - propanediol from glycerol as well. Commercial interests are exploring the production of 1,3 - propanediol from glycerol or glucose, in recombinant organisms which have been engineered to express the enzymes necessary for 1,3 - propanediol production from other organisms.

3 - hydroxypropionic acid CAS registry Number [503-66-2] (abbreviated as 3-HP) is a three carbon non-chiral organic molecule. The IUPAC nomenclature name for this molecule is propionic acid 3 - hydroxy. It is also known as 3 - hydroxypropionate, β - hydroxpropionic acid, β - hydroxypropionate, 3 - hydroxypropionic acid, 3 - hydroxypropanoate, hydracrylic acid, ethylene lactic acid, β -lactic acid and 2 - deoxyglyceric acid. Applications of 3-HP include the manufacture of absorbable prosthetic devices and surgical sutures, incorporation into beta-lactams, production of acrylic acid, formation of trifluromethylated alcohols or diols, polyhydroxyalkonates, and co-polymers with lactic acid. 3-HP for commercial use is now commonly produced by organic chemical syntheses. The 3-HP produced and sold by these methods is relatively expensive, and it would be cost prohibitive to use it for the production of monomers for polymer production. As discussed below, some organisms are known to produce 3-HP. However, there is not yet available a catalog of genes from these organisms and thus the ability to synthesize 3-HP using the enzymes natively responsible for the synthesis of that molecule in the native hosts which produce it does not now exist.

In addition to its commercial utility, 3-HP it is found in a number of biological processes, notably including many naturally occurring bio-polymers. Poly(3 - hydroxybutyrate) (PHB) is the most abundant member of the microbial polyesters which contain hydroxy monomers termed polyhydroxyalkonates (PHAs). PHB has utility as a biodegradable thermoplastic material and the material was first produced industrially in 1982.

The majority of published research on PHA's that contain 3-HP has concentrated on two bacterial sources: *Ralstonia eutropha ("Alcaligenes eutrophus")* and *Pseudomonas oleovorans.* Both *Ralstonia eutropha* and *Pseudomonas oleovorans* are able to grow on a nitrogen free media containing 3 - hydroxy - propionic acid, 1,5 - pentanediol or 1,7 - heptanediol. When 3-HP is the major hydroxy-acid added to the growth media, poly(3- hydroxybutyrate - co - 3 - hydroxypropionic acid) is formed containing 7 mol % 3 - hydroxypropionic acid. These cells also store 3 mol %, 3 - hydroxypropionic acid poly(3- butyrate - co - 3 - hydroxypropionic acid).

Recombinant systems have been used to create PHAs. An *E. coli* strain engineered to express PHA synthase from either *Ralstonia eutropha* or *Zoolgoea ramigera* produced poly(3 - hydroxypropionic acid) when feed 1,3 - propanediol. Skraly, F. A. "Polyhydroxyalkanoates Produced by Recombinant *E. coli."* Poster at Engineering Foundation Conference: Metabolic Engineering II. 1998. An *E. coli* strain that expressed PHA synthase (MBX820), when provided with the genes encoding glycerol dehydratase and 1,3 - propanediol dehydratase from *K. pneumonia ,* and 4 - hydroxybutyral- CoA transferase from *Clostridium kluyveri,* synthesized PHB from glucose.

Glycerol dehydratase, found in the bacterial pathway for the conversion of glycerol to 1,3 - propanediol, catalyzes the conversion of glycerol to 3 - hydroxypropionaldehyde and water. This enzyme has been found in a number of bacteria including strains of *Citrobacter, Klebsiella, Lactobacillus, Entrobacter* and *Clostridium.* In the 1,3 - propanediol pathway a second enzyme 1,3 - propanediol oxido-reductase (EC 1.1.202) reduces 3 - hydroxypropanaldehyde to 1,3 - propanediol in a NADH dependant reaction. The pathway for the conversion of glycerol to 1,3 - propanediol has been expressed in *E. coli.* Tong et al., Applied and Environmental Microbiology 57 (12) 3541-3546. The genes responsible for the production of 1,3 - propanediol were cloned from the *dha* regulon of *Klebsiella pneumoniae.* Glycerol is transported into the cell by the glycerol facilitator, and then converted into 3 - hydroxy - propionaldehyde by a coenzyme B₁₂- dependent dehydratase. *E. coli* lacks a native *dha* regulon, consequently E. *coli* cannot grow anaerobically on glycerol without an exogenous electron acceptor such as nitrate or fumarate.

Aldehyde dehydrogenases are enzymes that catalyze the oxidation of aldehydes to carboxylic acids. The genes encoding non-specific aldehyde dehydrogenases have been identified in a wide variety of organisms e.g.; *ALDH2* from *Homo sapiens, ALD4* from *Saccharomyces cerevisiae,* and from *E. coli* both *aldA* and *aldB,* to name a few. These enzymes are classified by co-factor usage, most require either AND⁺, or NADP⁺ and some will use either co-factor. The genes singled out for mention here are able to act on a number of different aldehydes and it likely that they may be able to oxidize 3 - hydroxy - propionaldehyde to 3 - hydroxypropionic acid.

### BRIEF SUMMARY OF THE INVENTION

The present invention is intended to permit the creation of a recombinant microbial host which is capable of synthesizing 3-HP from a starting material of glycerol or glucose. The glycerol or glucose is converted to 3 - hydroxypropionicaldehyde (abbreviated as 3-HPA) which is then converted to 3-HP. This process requires the so-called *dhaB* gene from *Klebsiella pneumoniae* which encodes the enzyme glycerol dehydratase and any one of four different aldehyde dehydrogenase genes to convert 3-HPA to 3-HP. The four aldehyde dehydrogenase genes used were *aldA* from the bacterium *E. coli, ALDH2* from humans, *ALD4* from the yeast *Saccharomyces cerevisiae, and aldB* from *E. coli.* The yeast gene appeared to give the best results.

The present invention provides a method for producing 3-hydroxypropionic acid comprising the steps of providing in a fermenter a recombinant microorganism which carries genetic constructions for the expression of a glycerol dehydratase and an aldehyde dehydrogenase, which are capable of catalyzing the production of 3-hydroxypropionic acid from glycerol, providing a source of glycerol or glucose for the recombinant microorganism and fermenting the microorganism under conditions which result in the accumulation of 3-hydroxypropionic acid.

The invention further provides a bacterial host comprising in its inheritable genetic material a genetic construction encoding for the expression of a glycerol dehydratase enzyme and an aldehyde dehydrogenase enzyme, such that the bacterial host is capable of converting glycerol to 3-hydroxypropionic acid.

The invention further provides, in a specific embodiment, a recombinant E. *coli* host comprising in its inheritable genetic materials the *dhaB* gene from *Klebsiella pneumoniae* and the *ald4* gene from *Saccharomycetes cerevisiae,* such that the host is capable of producing 3-hydroxypropionic acid from glycerol.

### DETAILED DESCRIPTION OF THE INVENTION

It is disclosed here that it is possible to introduce into a bacterial host genes encoding two enzymes and thus confer upon that host the ability to produce 3-HP from glycerol. The two necessary enzymes are glycerol dehydratase and aldehyde dehydrogenase. It is here reported that the two enzymes are both necessary and sufficient to enable a strain of a suitable host, such as a competent *E. coli* strain, to make 3-HP from glycerol. An exemplary gene encoding a glycerol dehydratase is known, the *dhaB* gene from *Klebsiella pneumoniae,* sequenced and rendered convenient to use. Several exemplary aldehyde dehydrogenases are known, and their sequences are presented here. From this information, it becomes practical to confer upon a bacterial host the ability to convert glycerol into 3-HP in a commercially reasonable manner.

It was not apparent before the completion of the work described here that these two diverse enzymes could be produced in a common host to produce the ability to make 3-HP. There are many known aldehyde dehydrogenase enzymes and genes, and the enzymes are known to have varying substrate specificities and efficiencies. There was not evidence, prior to the work described here, that the aldehyde dehydrogenase enzyme would work on the 3-hydroxypropionicaldehyde (3-HPA) substrate to create 3-HP. Without that knowledge, there was no data from which to predict the effectiveness of the 3-HP production studies described below. An additional uncertainty arises from the fact that the intermediate aldehyde, 3-HPA, is toxic to many bacterial host and thus the survival of the host is dependent upon the relative rates of enzymatic production and conversion of the aldehyde intermediate to non-toxic 3-HP.

A difficulty in the realization of the production of 3-HP desired here is that ribosome binding sites from non-native hosts are often ineffectual and lead to poor protein production and that many non-native promoters are often poorly transcribed and a bar to high protein expression. However, the inventors also recognized that a non-native promoter that is known to be very active and is inducible by the addition of a small molecule unrelated to the pathway being expressed is often a very efficient way to express and regulate the levels of enzymes expressed in hosts such as *E. coli.* To achieve high levels of regulated gene expression plasmids were constructed which placed the expression of all exogenous genes necessary for the production of 3 - hydroxypropionic acid from glycerol under the regulation of the *trc* promoter. The *trc* promoter, is efficient, not native to *E. coli,* and inducible by the addition of IPTG.

The present specification describes a genetic construct for use in the production of 3 - hydroxypropionic acid from glycerol. The genetic construct includes exemplary DNA sequences coding for the expression of a glycerol dehydratase and a DNA sequence coding for aldehyde dehydrogenase. The set of exemplary sequences necessary for the expression of glycerol dehydratase is collectively referred to as *"dhaB".* The set of sequences necessary for the expression of aldehyde dehydrogenase includes any one of four different genes which proved efficacious. The individual aldehyde dehydrogenase sequences referred to individually as *ALDH4, ALD2, aldA* and *aldB.*

### Producing 3 - hydroxypropionic acid in a foreign host

In the work described below, the enzymes necessary for the production of 3 - hydroxypropionic acid from glycerol in *E. coli* were expressed under the regulation of the *trc* promoter, a non-native promoter inducible by the addition of IPTG. The glycerol dehydratase was encoded by the *dhaB* gene from *Klebsiella pneumoniae,* the aldehyde dehydrogenases used was any one of four different genes *(ALDH2* from *Homo sapiens, ALD4* from *S. cerevisiae, aldB* from *E. coli* or *aldA* from *E. coli).* Expression of these genes coding for glycerol dehydratase and any one of the genes encoding an aldehyde dehydrogenases was sufficient to enable the construct to produce 3-HP when the fermentation media was supplemented with glycerol. In all of these constructs, the *dhaB* gene was downstream from the gene encoding the aldehyde dehydrogenase used, and expression of both genes was regulated by the *trc* promoter. This order, however, is not required and the order of the gens on a construct and the use of multiple constructs is possible.

In a minimal genetic construct made based on the data presented here, the only genetic elements present that would be necessary are the structural genes *dhaB* and an aldehyde dehydrogenase gene encoding a protein that efficiently catalyzes the oxidation of 3-hydroxypropionaldehyde to 3-hydroxypropionic acid, and non-native promoter sequences specifically selected to give the type of inducible control most appropriate for the context of the process in which the construct is to be used. Extraneous pieces of DNA, whether retained in the construct or added from other DNA sequences, would not necessarily be detrimental to effective 3-HP synthesis by the host organism, but would not be needed. Each sequence to be translated would necessarily be preceded by a ribosome binding site, functional in the selected host so that the messenger RNA(s) coding for the proteins of interest could be translated by ribosomes. Terminator sequences immediately downstream of each translated unit would also be necessary in some organisms, particularly in eukaryotes. The construct could be part of an autonomously replicating sequence, such as a plasmid or phage vector, or could be integrated into the genome of the host.

The structural genes and appropriate promoter(s) could be isolated by the use of restriction enzymes, by the polymerase chain reaction (PCR), by chemical synthesis of the appropriate oligonucleotides, or by other methods apparent to those skilled in the art or molecular biology. The promoter(s) would be derived from genomic DNA of other organism or from artificial genetic constructs containing promoters. Appropriate promoter fragments would be ligated into the construct upstream of the structural genes in any one of several possible arrangements.

The aldehyde dehydrogenase expressed would have: high specific activity towards 3-hydroxypropionaldehyde; be very stable in the host it is expressed in; be readily over expressed in the selected host; not be inhibited by either the substrates necessary for the reaction or the products formed by the reaction; be fully active under the fermentation conditions most favorable for the production of 3 - hydroxypropionic acid and be able to use either NAD⁺ or NADP⁺.

One possible arrangement is the true operon, where one promoter is used to direct transcription in one direction of all necessary Open Reading Frames (ORFs). The entire message is then contained in one messenger RNA. The advantages of the operon are that it is relatively easy to construct, since only one promoter is needed; that is it is relatively simple to replace the promoter with another promoter if that would be desirable later; and that it assures that the two genes are under the same regulation. The main disadvantage of the operon scheme is that the levels of the expression of the two genes cannot be varied independently. If it is found that the genes, for optimal 3 - hydroxypropionic acid synthesis, should be expressed at different levels, the operon in most cases cannot be used to realize this.

Another possible arrangement is the multiple-promoter scheme. Two or more promoters, with the same or distinct regulatory behavior, could be used to direct transcription of the genes. For example, one promoter could be used to direct transcription of *dhaB* and one to direct transcription of the gene encoding the appropriate aldehyde dehydrogenases. Because the genes theoretically can be transcribed and translated separately, a great number of combinations of multiple promoters is possible. Additionally, it would be most desirable to prevent the promoters from interfering with one another. This could be achieved either by placing two promoters into the construct such that they direct transcription in opposite directions, or by inserting transcriptional terminator sequences downstream of each separately transcribed unit. The main advantage of the multiple-promoter construct is that it permits independent regulation of as many distinct units as desired, which could be important. The disadvantages are that it would be more difficult to construct; more difficult to amend later; and more difficult to effectively regulate, since multiple changes in fermentation conditions would need to be introduced and might render the performance of the fermentation somewhat less predicable.

In any construct, the promoter sequence(s) used should be functional in the selected host organism and preferably provide sufficient transcription of the genes comprising the glycerol to 3 - hydroxypropionic acid pathway to enable the construct to be adequately active in that host. The promoter sequence(s) used would also effect regulation of transcription of the genes enabling the glycerol to 3-HP pathway to be adequately active under the fermentation conditions employed for 3-HP production, and preferably they would be inducible, such that expression of the genes could be modulated by the inclusion in, or exclusion from, the fermentation of a certain agents or conditions.

A plausible example of the use of such a construct follows: one promoter, which induced by the addition of an inexpensive chemical (the inducer) to the medium, could control transcription of both the d*haB* gene and the gene encoding the appropriate aldehyde dehydrogenase. The cells would be permitted to grow in the absence of the inducer until they accumulated to a predetermined level. The inducer would then be added to the fermentation and nutritional changes commensurate with the altered metabolism would be made to the medium as well. The cells would then be permitted to utilize the substrate(s) provided for 3-HP production (and additional biomass production if desired). After the cells could no longer use substrate to produce 3-HP, the fermentation would be stopped and the 3-HP recovered.

### Genetic Sequences

To express glycerol dehydratase and a suitable aldehyde dehydrogenase, the two enzymes necessary for the production of 3 - hydroxypropionic acid from glycerol, it is required that the DNA sequences containing the glycerol dehydratase and aldehyde dehydrogenase coding sequences be combined with at least a promoter sequence (preferably a non-native promoter although some native promoter activity may be present). An exemplary method of construction is described in the example below. To ensure that the present specification is enabling, the full sequences of the coding regions of genes for these enzymes is presented here.

Sequences 1, 3, 5 and 7 present different native genomic sequences for genes encoding aldehyde dehydrogenases.

SEQ ID NO:1 contains the full native DNA sequence encoding the *ALD4* enzyme from *Saccharomyces cerevisiae.* The amino acid sequence of the protein is presented as SEQ ID NO:2.

SEQ ID NO:3 includes the DNA sequence for the human *ALDH2* gene, again including the full protein coding region. The amino acid sequence for this human alcohol dehydrogenase is presented in SEQ ID NO:4.

SEQ ID NO:5 and 7 respectively present the full coding sequences from the E. coli genes aldA and aldB, both of which encode alcohol dehydrogenases. The amino acid sequences for the proteins encoded by the genes are presented in SEQ ID NO: 6 and 8 respectively.

SEQ ID NO:9 contains the native genomic DNA sequence for the *dhaB* gene from the *dha* regulon of *Klebisiella pneumoniae.* The coding sequences for this complex regulon produces five polypeptides, which are presented as SEQ ID NOS:10 through 13, which together provide the activity of the glycerol dehydratase enzyme.

Each of these coding sequences can be used to make genetic constructs for the expression of the appropriate enzymes in a heterologous hosts. In making genetic constructs for expression of the genes in such hosts, it is contemplated that heterologous promoters will be joined to the coding sequences for the enzymes, but all that it required is that the promoters be effective for the hosts in which the genes are to be expressed. It is also contemplated and envisioned that significant variations in DNA sequence are possible from the native DNA coding sequences presented here. As is well known in the art, due to the degeneracy of the genetic code, many different DNA sequences can encode the expression of the same protein. So, when this document uses language specifying a DNA sequence encoding a protein, it is intended to encompass any DNA sequence which can be used to express that protein even if different from the genomic sequences presented here. It is also contemplated that conservative changes in the amino acid sequences of the proteins specified here can be made without departing from the present invention. In particular, deletions, additions and substitutions of one or more amino acids in a protein sequence can almost always be made without changing protein functionality. When the name of a protein is sued here, it is intended to be equally applicable to both such minor changes in amino acid sequence and to allelic variations in native protein sequence as occurs within the species named as well as other closely related species.

It is possible that many of the above DNA sequences could be truncated and still express a protein that has the same enzymatic properties. One skilled in the art of molecular biology would appreciate that minor deletions, additions and mutations may not change the attributes of the designated base pair sequences; many of the nucleotide of the designated base pair sequences are probably not essential for their unique function. To determine whether or not an altered sequence or sequences has sufficient homology with the designated base pairs to function identically, one would simply create the candidate mutation, deletion or alteration and create a gene construct including the altered sequence together with promoter and termination sequences. This gene construct could be tested as, described below, for the production of 3-HP from glycerol.

Certain DNA primers were used to isolate or clone the genomic DNA sequences used in the experiments described below. While the sequence information presented here is sufficient to enable the construction of expression plasmids incorporating the genes identified here, in order to redundantly enable the use of these genes, primers which may be used to isolated the genes from their native hosts are described below.

The primers aldA_L (SEQ ID NO:14), and aldA_R (SEQ ID NO:15), were used to amplify the 1513 bp *ald*A fragment from genomic *E. coli* DNA (strain MG1655, a gift from the Genetic Stock Center, New Haven, CT). The gel purified PCR fragment containing a DNA sequence coding for the expression of aldehyde dehydrogenase was inserted into *Nco*I*-Xho*I site of pSE380 (Invitrogen, San Diego, CA) to give pPFS3. The resulting plasmid contained *aldA* under the control of the *trc* promoter. This construct allowed for high-level expression of the *aldA* gene from *E. coli* under regulation of the *trc* promoter. Unless indicated otherwise all molecular biology and plasmid constructions were done in *E. coli* AG1 (Stratagene, La Jolla, CA).

The primers aldB_L (SEQ ID NO:20) and aldB_R (SEQ ID NO:21), were used to amplify the 1574 bp *aldB* fragment from genomic *E. coli* DNA (strain MG1655). The resulting PCR converted the TGA stop codon into a TAA stop codon. The gel-purified PCR fragment containing the DNA sequence sufficiently coding for the expression of aldehyde dehydrogenase was inserted into the *KpnI-SacI* site of pSE380 to give pPFS12.

The primers ALD4_L (SEQ ID NO : 16), and ALD4_R (SEQ ID NO : 17), were used to amplify the 1595 bp ALD4 fragment from S. *cerevisiae* DNA (strain YPH500). The gel-purified fragment containing a DNA sequence coding for the expression of aldehyde dehydrogenase was inserted into the *Kpn*I*-Sac*I site of pPFS3 to give pPFS8. The resulting plasmid contained mature *ALD4* under control of the *trc* promoter.

The primers ALDH2_L (SEQ ID NO:18), and ALDH2_R (SEQ ID NO:19), were used to amplify the 1541 bp ALDH2 fragment from pT7-7::ALDH2, a gift from H. Weiner (Purdue University, West Lafayette, IN). The gel purified PCR fragment containing a DNA sequence sufficiently homologous to base pairs 22 to 1524, inclusive of SEQ ID NO : 3 so as to code for the expression of aldehyde dehydrogenase was inserted in to the *Kpn*I*-Sac*I site of pSE380 to give pPFS7. This sequence was moved from pPFS7 into the *Kpn*I*-Sac*I site of pPFS3 to give pPFS9. The resulting plasmid contained mature ALDH2 under the control of the *trc* promoter.

The primers pTRC_L (SEQ ID NO:22), and pTRC_R )SEQ ID NO:23), were used to amplify the 540 bp fragment from pSE380. The gel purified PCR fragment was inserted into the *Hpa*I-*Kpn*I site of pPFS3 to give pPFS13. The resulting plasmid deleted the "native" ribosome binding site of pSE380 and a *Nco*I site (which contained an extraneous ATG start codon upstream of the cloned genes). The *Kpn*I-*Sac*I fragments of pPFS8, pPFS9, and pPSF12 were inserted into the *Kpn*I-*Sac*I site of pPFS13 to give pPFS14, pPFS15, and pPFS16, respectively.

### Assay for production of 3-HP

The efficacy of changes made as contemplated herein can be checked by the following tests. To test for the production of 3-HP, fermentation products can be quantified with a Waters Alliance Integrity HPLC system (Milford, MA) equipped with a refractive index detector, a photodiode array detector, and an Aminex HPX-87H (Bio-Rad, Hercules, CA) organic acids column. The mobile phase should be 0.01 N sulfuric acid solution (pH 2.0) at a flow rate of 0.5 mL/min. The column temperature should be set to 40°C. Compounds can be identified by determining if they co-elute with authentic standards. Prior to analysis, all samples should be filtered through 0.45 µM pore size membrane. (Gelman Sciences, Ann Arbor, MI). The fractions of the fermentation products collected using HPLC should be analyzed on a Varian Star 3400 CX, gas - chromatograph coupled to a Varian Saturn 3 mass spectrometer (GC-MS) (Walnut Creek, CA).

### Assay for enzyme activity.

Aldehyde dehydrogenase activity can be determined by measuring the reduction of β-NAD+ at 25°C with 3 - hydroxypropionaldehyde as a substrate. All buffers should contain 1 mM ethylenediaminetetraacetic acid (EDTA), 0.1 mM Pefabloc SC (Boehringer Mannheim, Indianapolis, IN) and 1 mM Tris (carboxyethyl) phosphine hydrochloride (TCEP-HCL). For ALD4, the solution should contain 100 mM Tris HCL Buffer (pH 8.0), 100 mM KCl. For ALDH2 the solution should contained 100 mM sodium pyrophosphate (pH 9.0). For AIdA and AldB, the solution should contain 20 mM sodium glycine (pH 9.5). A total of 3.0 mL of buffer should be added to quartz cuvettes and allowed to equilibrate to assay temperature. From 5 to 20 µL of cell extract should be added and background activity recorded after the addition of β-NAD⁺ to a final concentration of 0.67 mM. The reaction should be started by the addition of substrate (either acetaldehyde, propionaldehyde, or 3 - hydroxypropionaldehyde) to a final concentration of 2 mM. Assay mixtures should be stirred with micro-stirrers during the assays.

For aldehyde dehydrogenase activity assays, one unit is defined as the reduction of 1.0 µM of β-AND⁺ per minute at 25 °C. These reactions can be monitored by following the change in absorbence at 340 nm (A₃₄₀) at 25°C on a Varain Carry-1 Bio spectrophotometer (Sugar Land, TX). Total protein concentrations in the cell extracts can be determined using the Bradford assay method (Bio-Rad, Hercules, CA) with bovine serum albumin as the standard.

### EXAMPLES

### Plasmid constructions.

*Klebsiella pneumoniae* expresses glycerol dehydratase, an enzyme that catalyzes the conversion of glycerol to 3 - hydroxypropionaldehyde, *(dhaB)* and 1,3 - propanediol oxidoreductase an enzyme that catalyzes the conversion of 3 - hydroxypropionaldehyde to 1,3 - propanediol respectively (the gene product from *dhaT).* A plasmid encoding these two genes was created and expressed in *E. coli* (plasmid pTC53). The *dhaT* gene was deleted from pTC53 to create pMH34. The resulting plasmid still contained the DNA sequence complementary to base pairs 330 to 2153 inclusion of SEQ ID NO : 9, the complement of base pairs 2166 to 2591, inclusive, of SEQ ID NO : 9, and the complement of base pairs 3191 to 4858, inclusive, of SEQ ID NO : 9, so as to code for the expression of glycerol dehydratase. The fragment of DNA encoding these sequences was excised from pMH34 by cutting it with *Sall-Xbal, and* the resulting fragment was gel purified (the purified fragment was gift from M. Hoffman of the University of Wisconsin - Madison). This DNA fragment was inserted into the *Sal*I*-Xba*I site of pPFS13 to give pPFS17.

The resulting plasmid contained both the *aldA* and *dhaB* genes under the control of the *trc* promoter. Similarity, the gel-purified *Sal*I*-Xba*I fragment from pMH34 was inserted into the *Sal*I*-Xba*I sites of pPFS14, pPFS15, and pPFS16 to give pPFS18, pPFS 19, and pPFS20, respectively. These plasmids contained *ALD4, ALDH2,* and *aldB,* respectively, as well as *dhaB* under the control of the *trc* promoter; in all of the constructs the *dhaB* gene were downstream of the gene encoding the aldehyde dehydrogenase.

### Expression in E. coli.

The efficacy of E. *coli* as a platform for the production of 3-HP from growth on glucose has been examined using a mathematical model developed for this purpose. The model was executed in two different ways assuming the conversion of one mole of glucose under either anaerobic or aerobic conditions either directly to 3-HP or to the production of 3-HP and ATP. The optimum yield under anaerobic conditions is 1 mole of 3-HP and 1 mole of lactate. The more realistic yield under anaerobic conditions is 0.5 moles of 3-HP, 1.5 moles of lactate and 1 mole of ATP. The optimum yield under aerobic conditions is 1.9 moles of 3-HP and 0.3 moles of CO₂, The realistic yield under aerobic conditions is 1.85 moles of 3-HP, 0.35 moles of CO₂ and 1 mole of ATP.

The effect of 3-HP concentration on *E. coli* strain MG1655 growth was measured. Cells were grown on standard media with and without the addition of up to 80g/L of 3-HP. The best fit of these data demonstrated that 3-HP was only 1.4 times as inhibitory as lactic acid on the growth of *E*. *coli.* It is possible to economically produce lactic acid using *E. coli,* since 3-HP is only 1.4 times more inhibitory than lactic acid, it should be possible to use *E*. *coli* as a host for the commercial production of 3-HP.

### Media and growth conditions

The standard media contained the following per liter: 6 g Na₂BPO₄, 3 g KH₂PO₄, 1 g NH₄Cl, 0.5 g NaCl, 3 mg CaCl₂, 5 g yeast extract (Difco Laboratories, Detroit, MI) and 2 mM MgSO₄. When necessary to retain plasmids ampicillin (100 mg/mL) was added to the media. Isopropyl-β-thiogalactopyranoside (IPTG) was added in varying amounts to induce gene expression. All fermentations were carried out in an incubator-shaker at 37 C and 200 rpm. Anaerobic fermentations were carried out in 500-mL anaerobic flasks with 300 mL of working volume. Inocula for fermentations were grown overnight in Luria-Bertani medium supplemented with ampicillin is necessary. The 300-mL fermentations were inoculated with 1.5 mL of the overnight culture. For enzyme assays, fermentations were incubated for 24 hours.

### Over expression of aldehyde dehydrogenase in E. coli.

Cells were harvested by centrifugation at 3000 x g for 10 minutes at 4°C with a Beckman (Fullerton, CA) model J2-21 centrifuge. Cell pellets were washed twice in 100 mM potassium phosphate buffer at pH 7.2 and re-suspended in appropriate assay resuspension buffer equal to 5 x of the volume of the wet cell mass. The cells were homogenized using a French pressure cell. The homogenate was centrifuged at 40000 x g for 30 minutes. The supernatant was dialyzed against the appropriate resuspension buffer using 10000 molecular weight cut-off pleated dialysis tubing (Pierce, Rockford, IL) at 4°C. Dialysis buffer was changed after 2 hours, and 4 hours, and dialysis was stopped after being allowed to proceed overnight.

*E. coli* AG1 cells transfected with the plasmids constructed to express the *alda, ALD4, ALDH2,* or *aldB* genes were grown in 500-mL anaerobic flasks. Twelve hours after the fermentations were inoculated IPTG was added to induce enzyme expression. The cells were allowed to grow for an additional 12 hours then harvested and lysed as discussed above. The soluble fraction of the lysate was assayed for aldehyde dehydrogenase activity using the substrate 3-hydroxypropionicaldehyde in the buffer appropriate for the particular enzyme expressed The plasmid, aldehyde dehydrogenase expressed and specific activity measured (U/mg of protein) were as follows: pPFS13, *aldA,* 0.2; pPFS14, *ALD4,* 0.5, pPFS15, *ALDH2,* 0.3; and pPFS16, *aldB.* 0.1. The control, *E. coli* strain AG1 harboring plasmid pSE380, encoded no exogenous aldehyde dehydrogenase activity and it had no detectable activity with 3-HP as substrate. It is clear from the activity assays that all four aldehyde dehydrogenases were expressed in *E. coli.* The aldehyde dehydrogenase cloned from *Saccharomyces cerevisiae (ADH4)* had the highest activity when 3-hydroxypropionaldehyde was used as the substrate (0.5 units/mg of protein).

*E. coli* cells transformed with plasmids expressing: aldehyde dehydrogenase; both aldehyde dehydrogenase and glycerol dehydratase, or neither gene; were grown and assayed for their ability to produce 3-HP from glycerol. The cells were grown on standard media supplemented with 6 µM of Coenzyme B₁₂, under anaerobic conditions in the absence of light (to protect the integrity of the Coenzyme B₁₂ necessary for DhaB activity). After 12 hours, IPTG was added to induce expression of the genes under the *trc* promoter at the same time 5g/L of glycerol was added. After 12 more hours of anaerobic fermentation the fermentation broth was assayed for 3 - HP by HPLC and GC, the plasmid, aldehyde dehydrogenase gene expressed and g/L of 3- HP measured were as follows: pSF17, *aldA,* 0.031; pPSF 18 *ALD4,* 0.173; and pPSF 19, *ALDH2,* 0.061. Cells expressing *dhaB* but no exogenous aldehyde dehydrogenase genes (plasmid pMH34) produced 0.015 g/L of 3 - HP. Cells expressing *aldA, ALD4, ALDH2* or *aldB* but not *dhaB* (plasmids pPFS13, pPFS14, pPFS15, pPFS16, respectively) all produced less then 0.005 g/L of 3-HP when the media the cells were growing in was supplemented with 2.5g/L of 3-hydroxypropionaldehyde.

### Other Hosts and Promoters

Applications of the 3 - hydroxypropionic acid pathway such as the genetic constructs of the present invention can easily be expressed in other organisms. The required genes would need to be placed under control of an appropriate promoter or promoters. Some organism such as yeasts may require transcription terminators to be placed after each transcribed unit. The knowledge of the present intention makes such amendments possible. Such a genetic construct would need to be part of a vector that could either replicate in the new host or integrate into the chromosome of the new host. Many such vectors are commercially available for expression in gram-negative and gram-positive bacteria, yeast, mammalian cells, insect cell, plant, etc. For example, to express the 3-hydroxypropionic acid pathway in *Rhodobacter capsulatus,* one could obtain vector pNH2 from the American Type Culture Collection (ATTC). This is a shuttle vector for use in *R. capsulatus* and *E. coli.* Organisms such as *Saccharomyces cerevisiae* which can convert glucose to glycerol could be used as a host, such a construct would enable the production of 3 - HP directly from glucose. Additionally, other substrates such as xylan could also be used given the selection of an appropriate host.

Stochiometric analysis shows that best stochiometric yield of 3-HP production in *E. coli* calculated on the basis of glucose consumed is obtained under aerobic conditions. Under aerobic condition CO₂ is the only carbon-containing co-product, in particular the generation of lactic acid which occurs under anaerobic conditions is avoided. Production of 3-HP under these conditions could result in a more economical recovery of 3-HP from the fermentation broth.

Alternatively, the *dhab* gene and a gene encoding the appropriate aldehyde dehydrogenase could be cloned into the multiple cloning site of this vector in E. *coli* to facilitate construction, and then transformed into *R. capsulatus.* The *R. capsulatus* nifH promoter, provided on the plasmid, could be used to direct the transcription in R. *capsulatus* of the genes placed into pNF2 in series with one promoter, or with two copies of the nifH promoter. Expression of the genes in other organisms would require a procedure analogous to that presented here.

### Alternative Aldehyde Dehydrogenases and Glycerol Dehydratases

Applications of the pathway for the production of 3-hydroxypropionic acid from glycerol can be made using other suitable aldehyde dehydrogenases. To be functional in this pathway an aldehyde dehydrogenase needs to be stable, readily expressed in the host of choice and have high enough activity towards 3-hydroxypropionaldehyde to enable it to make 3-HP. The knowledge of the present invention makes such amendments possible. A program of directed evolution could be undertaken to select for suitable aldehyde dehydrogenases or they could be recovered from native sources, the genes encoding these enzymes in conjunction with a gene encoding an appropriate glycerol dehydratase activity, would then be made part of any of the constructs envisioned here to produce 3 - hydroxypropionic acid from glycerol.

A similar program of enzyme improvement including for example directed evolution could be carried out using the *dhaB* gene from *Klebsiella pneumoniae* as a starting point to obtain other variants of glycerol dehydratase that are superior in efficiency and stability to the form used in this invention. Alternatively, enzymes which catalyzes the same reaction may be isolated from others organisms and used in place of the *Klebsiella pneumoniae* glycerol dehydratase. Such enzymes may be especially useful in alternative hosts wherein they may be more readily expressed, be more stable and more efficient under the fermentation conditions best suited to the growth of the construct and the production and recovery of 3-HP.

## Claims

1. A method for producing 3-hydroxypropionic acid comprising the steps of providing in a fermenter a recombinant microorganism which carries genetic constructions for the expression of a glycerol dehydratase and an aldehyde dehydrogenase, which are capable of catalyzing the production of 3-hydroxypropionic acid from glycerol;
providing a source of glycerol or glucose for the recombinant microorganism, and
fermenting the microorganism under conditions which result in the accumulation of 3-hydroxypropionic acid.

2. The method of claim 1, wherein the recombinant microorganism carries a genetic construct which expresses the *dhaB* gene from *Klebsiella pnewnoniae* and a gene for an aldehyde dehydrogenase.

3. The method of claim 1 or 2, wherein the gene for the aldehyde dehydrogenase is selected from the group consisting of *ALDH2, ALD4, aldA* and *aldB.*

4. The method of claim 1 or 2, wherein the aldehyde dehydrogenase is selected from the group consisting of SEQ ID NO: 2, SEQ ID NO: 4, SEQ ID NO: 6 and SEQ ID NO: 8.

5. The method of any one of the preceding claims, wherein the microorganism is *E. coli.*

6. A bacterial host comprising in its inheritable genetic material a genetic construction encoding for the expression of a glycerol dehydratase enzyme and an aldehyde dehydrogenase enzyme, such that the bacterial host is capable of converting glycerol to 3-hydroxypropionic acid.

7. The bacterial host of claim 6 which is a recombinant *E. coli* host.

8. The bacterial host of claim 6 or 7, wherein the glycerol dehydratase is from *Klebsiella pneumoniae.*

9. The bacterial host of claim 8, wherein the gene encoding the glycerol dehydratase is the *dhaB* gene from *Klebsiella pneumoniae.*

10. The bacterial host of any one of claims 6 to 9, wherein the aldehyde dehydrogenase is selected from the group consisting *of ALDH2, ALD4, aldA* and *aldB.*

11. The bacterial host of any one of claims 6 to 9, wherein the aldehyde dehydrogenase is selected from the group consisting of SEQ ID NO: 2, SEQ ID NO:4, SEQ ID NO: 6 and SEQ ID NO: 8.

12. A recombinant *E. coli* host comprising in its inheritable genetic materials the *dhaB* gene from *Klebsiella pneumoniae* and the *ald4* gene from *Saccharomycetes cerevisiae,* such that the host is capable of producing 3-hydroxypropionic acid from glycerol.

## Patentansprüche

1. verfahren zur Herstellung von 3-Hydroxypropionsäure, umfassend die folgenden Stufen:
das Bereitstellen eines rekombinanten Mikroorganismus, der genetische Konstruktionen für die Expression einer Glycerin-dehydratase und einer Aldehyd-dehydrogenase trägt, die zur Katalyse der Produktion von 3-Hydroxypropionsäure aus Glycerin befähigt sind, in einem Fermenter;
das Bereitstellen einer Quelle für Glycerin oder Glucose für den rekombinanten Mikroorganismus und
das Fermentieren des Mikroorganismus unter Bedingungen, die zur Anreicherung von 3-Hydroxypropionsäure führen.

2. Verfahren nach Anspruch 1, wobei der rekombinante Mikroorganismus ein genetisches Konstrukt trägt, das das dhaB-Gen aus Klebsiella pneumoniae und ein Gen für eine Aldehyd-dehydrogenase exprimiert.

3. Verfahren nach Anspruch 1 oder 2, wobei das Gen für die Aldehyd-dehydrogenase aus der Gruppe ALDH2, ALD4, aldA und aldB ausgewählt ist.

4. Verfahren nach Anspruch 1 oder 2, wobei die Aldehyd-dehydrogenase aus der Gruppe SEQ ID NO:2, SEQ ID NO:4, SEQ ID NO:6 und SEQ ID NO:8 ausgewählt ist.

5. Verfahren nach einem der vorstehenden Ansprüche, wobei es sich bei dem Mikroorganismus um E. coli handelt,

6. Bakterieller Wirt, umfassend in seinem vererbbaren genetischen Material eine genetische Konstruktion, die für die Expression eines Glycerin-dehydxatase-Enzyms und eines Aldehyd-dehydrogenase-Enzyms kodiert, so dass der bakterielle Wirt zur Umwandlung von Glycerin in 3-Hydroxypropionsäure befähigt ist.

7. Bakterieller Wirt nach Anspruch 6, wobei es sich um einen rekombinanten E. coli-Wirt handelt.

8. Bakterieller Wirt nach Anspruch 6 oder 7, wobei die Glycerin-dehydratase aus Klebsiella pneumoniae stammt.

9. Bakterieller Wirt nach Anspruch 8, wobei es sich bei dem Gen, das für Glycerin-dehydratase kodiert, um das dhaB-Gen aus Klebsiella pneumoniae handelt.

10. Bakterieller Wirt nach einem der Ansprüche 6 bis 9, wobei die Aldehyd-dehydrogenase aus der Gruppe ALDH2, ALD4, aldA und aldB ausgewählt ist.

11. Bakterieller Wirt nach einem der Ansprüche 6 bis 9, wobei die Aldehyd-dehydrogenase aus der Gruppe SEQ ID NO:2, SEQ ID NO:4, SEQ ID NO:6 und SEQ ID NO:8 ausgewählt ist.

12. Rekombinanter E. coli-Wirt, umfassend in seinem vererbbaren genetischen Material das dhaB-Gen aus Klebsiella pneumoniae und das ald4-Gen aus Saccharomycetes cerevisiae, so dass der Wirt zur Erzeugung von 3-Hydroxypropionsäure aus Glycerin befähigt ist.

## Revendications

1. Procédé de production d'acide 3-hydroxy-propionique, qui comporte les étapes suivantes :
- mettre dans un fermenteur un microorganisme génétiquement modifié, doté de constructions génétiques dédiées à l'expression d'une glycérol déshydratase et d'une aldéhyde déshydrogénase qui sont capables de catalyser la production d'acide 3-hydroxy-propionique à partir de glycérol,
- y mettre une source de glycérol ou de glucose pour le microorganisme génétiquement modifié,
- et faire fermenter ce microorganisme dans des conditions qui conduisent à l'accumulation d'acide 3-hydroxy-propionique.

2. Procédé conforme à la revendication 1, dans lequel le microorganisme génétiquement modifié est doté d'une construction génétique qui exprime le gène *dhaB* de *Klebsiella pneumoniae* et un gène d'aldéhyde déshydrogénase.

3. Procédé conforme à la revendication 1 ou 2, dans lequel le gène d'aldéhyde déshydrogénase est choisi dans l'ensemble formé par *ALDH2, ALD4, aldA* et *aldB.*

4. Procédé conforme à la revendication 1 ou 2, dans lequel l'aldéhyde déshydrogénase est choisie dans l'ensemble formé par les Séquences n° 2, n° 4, n° 6 et n° 8.

5. Procédé conforme à l'une des revendications précédentes, dans lequel le microorganisme appartient à l'espèce *E*. *coli.*

6. Hôte bactérien contenant, dans son matériel génétique héritable, une construction génétique codant l'expression d'une enzyme glycérol déshydratase et d'une enzyme aldéhyde déshydrogénase, de sorte que cet hôte bactérien est capable de convertir du glycérol en acide 3-hydroxy-propionique.

7. Hôte bactérien conforme à la revendication 6, qui est un hôte *E*. *coli* génétiquement modifié.

8. Hôte bactérien conforme à la revendication 6 ou 7, chez lequel la glycérol déshydratase est originaire de l'espèce *Klebsiella pneumoniae.*

9. Hôte bactérien conforme à la revendication 8, chez lequel le gène codant la glycérol déshydratase est le gène *dhaB* de *Klebsiella pneumoniae.*

10. Hôte bactérien conforme à l'une des revendications 6 à 9, dans lequel l'aldéhyde déshydrogénase est choisi dans l'ensemble formé par *ALDH2, ALD4, aldA* et *aldB.*

11. Hôte bactérien conforme à l'une des revendications 6 à 9, dans lequel l'aldéhyde déshydrogénase est choisie dans l'ensemble formé par les Séquences n° 2, n° 4, n° 6 et n° 8.

12. Hôte *E. coli* génétiquement modifié, contenant, dans son matériel génétique héritable, le gène *dhaB* de *Klebsiella pneumoniae* et le gène *ald4* de *Saccharomyces cerevisiae,* de sorte que cet hôte est capable de produire de l'acide 3-hydroxy-propionique à partir de glycérol.
